(19) Europäisches Patentamt
European Patent Office
Office européen des brevets

(11) **EP 3 019 151 B1**

(12) **EUROPEAN PATENT SPECIFICATION**

(45) Date of publication and mention of the grant of the patent:
**12.08.2020 Bulletin 2020/33**

(21) Application number: **14818467.4**

(22) Date of filing: **27.06.2014**

(51) Int Cl.:
***A61Q 5/06*** (2006.01) ***A61K 8/81*** (2006.01)
***A61K 8/87*** (2006.01)

(86) International application number:
**PCT/US2014/044610**

(87) International publication number:
**WO 2014/210480 (31.12.2014 Gazette 2014/53)**

(54) **COMPOSITIONS AND METHODS FOR TREATING HAIR**

ZUSAMMENSETZUNGEN UND VERFAHREN ZUR BEHANDLUNG VON HAAR

COMPOSITIONS ET MÉTHODES DE TRAITEMENT CAPILLAIRE

(84) Designated Contracting States:
**AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2013 US 201313931238**
**28.06.2013 US 201313931276**
**28.06.2013 US 201313931329**

(43) Date of publication of application:
**18.05.2016 Bulletin 2016/20**

(73) Proprietor: **L'OREAL**
**75008 Paris (FR)**
Designated Contracting States:
**AT BE BG CH CY CZ DE DK EE ES FI FR GB GR HR HU IE IS IT LI LT LU LV MC MK MT NL NO PL PT RO RS SE SI SK SM TR**

(72) Inventors:
- **TAN, Siliu**
**Westfield, New Jersey 07090 (US)**
- **SIMONNET, Jean-Thierry**
**92500 Rueil Malmaison (FR)**
- **SINGER, Jim**
**South Orange, New Jersey 07079 (US)**
- **NGUYEN, Nghi Van**
**Edison, New Jersey 08820 (US)**

(74) Representative: **Casalonga**
**Casalonga & Partners**
**Bayerstraße 71/73**
**80335 München (DE)**

(56) References cited:
**EP-A1- 2 570 192** **WO-A1-2013/074210**
**WO-A2-2010/133658** **WO-A2-2011/137338**
**FR-A1- 2 889 943** **US-A- 4 985 239**
**US-A1- 2003 026 815** **US-B1- 6 585 965**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 019 151 B1

## Description

## Cross-Reference to Related Applications

## Technical Field

The disclosure relates to hair styling compositions comprising at least two latex polymers, wherein at least one latex polymer is a film-forming polymer. In various embodiments of the disclosure, the at least two latex polymers are chosen to have certain properties. Compositions comprising the at least two latex polymers may, according to certain embodiments, form films that have surprising properties. Methods of styling the hair with such compositions are also disclosed.

## Background

Compositions for styling the hair are known, such as, for example, hair spray compositions, hair gels and mousses, hair volumizing compositions, hair smoothing creams, lotions, serums, oils, clays, etc. The goals of many hair styling compositions include to hold or fix the hair in a particular shape, to impart or increase volume of the hair, and/or to smooth the hair, e.g. to decrease or eliminate the appearance of frizz.

Drawbacks associated with current products for styling the hair include that the product is often sticky or tacky and/or often produces a film that imparts a sticky or tacky feel, and styled hair that is stiff and/or "crunchy" (i.e. the film is hard and brittle resulting in a crunching feel or sound when the hair is touched), which is undesirable for most consumers.

Current products for styling the hair typically include water soluble film-forming polymers. Depending on the chemical make-up of these polymers, they may be either soluble in water, or they may be water insoluble polymers which are made water soluble via various chemical modifications, such as neutralization. Solutions comprising these polymers tend to be viscous, i.e. as the concentration of the polymer increases, its viscosity builds up rapidly. Translated to styling applications, as the solvent evaporates, the polymer solution becomes thicker on the hair surface, resulting in a sticky or tacky film. These products also tend to exhibit problems with product spreadability, hair manageability, and low degree of humidity resistance which is particularly a problem in hot and humid countries.

The use of latex polymers is also known, for example, to provide extended-wear properties to a cosmetic product (e.g. mascara, eyeliner, nail polish) into which they are formulated.

Some known compositions include one latex polymer. For example, U.S. Patent No. 6,126,929 describes a composition comprising a dispersion of a latex film former, optionally with a plasticizer, and a non film-forming particle not capable of being film-formed. U.S. Patent No. 4,710,374 describes a composition comprising cationic polymers, a surfactant, and an anionic latex. U.S. Patent No. 7,740,832 describes a composition comprising at least one non-latex polymer and an anionic, cationic or amphoteric fixing polymer. U.S. Patent No. 4,798,721 describes a composition comprising a latex particle. U.S. Patent Application No. 2005/0089490 A1 describes a composition comprising a water-dispersible styling polymer and a gel-forming polymer.

Other known cosmetic compositions include various components to provide improved properties such as adhesion, flexibility, and compatibility of other components. For example, U.S Patent Application No. 2007/0224140 A1 describes a composition comprising a cosmetically acceptable medium, a non film-forming microsphere to provide adhesion, and a film-forming component comprising two water-borne emulsion polymers. French Patent Application No. FR 2 968 978A describes an eyeliner composition comprising at least two film-forming latexes and a plasticizer to increase the flexibility of the film. French Patent Application No. FR 2 898 050A describes a composition comprising a fatty acid ester, and a copolymer of a (meth)acrylate polymer and a hydroxyester (meth)acrylate. U.S. Patent Application No. 2009/0297467A describes a composition comprising at least one neutralized sulfonated polymer and mixtures of acrylates and hydroxyester acrylates. U.S. Patent Application No. 2009/035335 A1 describes a mascara composition comprising two water-dispersible acrylate polymers, and a cross-linked polymeric film-former to enhance the compatibility and bind the two water-dispersible acrylate polymers. International Patent Application No. WO 2011/137338 A2 describes a composition comprising a polyurethane dispersion and an acrylic film-forming dispersion. U.S. Patent Application No. 2004/0071646A describes an aerosol device containing a composition comprising a polyurethane dispersion having a particle size of from 0.1 - 1 μm, and at least one non-latex fixing polymer.

Additionally, some cosmetic compositions incorporate polymers having a core-shell structure. For example, U.S. Patent Application No. 2003/0064045 A1 describes a mascara composition comprising a dispersion of particles having a core-shell structure. U.S. Patent Application No. 2007/0286833 A1 describes a multistage polymer comprising a latex core-shell particle comprising a soft polymer and a hard polymer. In addition, U.S. Patent Application No. 2009/0317432A describes an applicator for makeup containing a composition comprising a colorant and at least one latex or core-shell latex particle.

Cosmetic compositions in a non-aqueous medium are known. For example, European Patent Application No.

EP 1 082 953A describes a dispersion comprising two film formers in isododecane. International Patent Application No. WO11056332A describes a composition comprising three volatile solvents, and at least one film former, for example silicon acrylate or acrylate, soluble or dispersible in at least one of the three solvents.

Compositions for use in mascaras may have low glass transition temperatures ("Tg") to obtain a soft film. For example, U.S. Patent Application No. 2010/0028284 A1 describes a mascara composition comprising at least two acrylate film formers, where the glass transition temperature ("Tg") of the mascara composition is ≤ 20°C. U.S. Patent Application No. 2006/134043A describes a mascara composition comprising a fatty acid and at least one acrylate resin emulsion.

Some known compositions use solubilized polymers rather than polymer particles. For example, U.S. Patent No. 7,651,693 describes a composition comprising a solubilized blend of two polymers. U.S. Patent No. 6,214,328 describes a composition comprising at least one acrylate latex that is soluble in solutions containing low volatile organic compounds or in water upon neutralization.

U.S. Patent No. 5,441,728 describes a composition comprising a water-soluble fixative polymer and a latex particle. Water-soluble polymers tend to be sticky, and may not be suitable for applications requiring a clean touch.

French Patent Application No. FR 2 834 458A describes a nail polish composition comprising two film formers in an aqueous medium in a specific ratio.

However, it has now been discovered that by providing a composition comprising at least two latex polymers, wherein at least one of said latex polymers is a film-forming polymer, it is possible to form a film on a substrate that has certain desirable properties, such as a clean, natural, and/or "invisible" feel, and a lack of stickiness. Such compositions may be useful in hair-styling applications wherein styling benefits such as natural look, curling or straightening, and styling hold are imparted to hair.

Moreover, compositions according to embodiments of the disclosure may be prepared that deliver a surprisingly broad range of hair styling benefits, such as, for example, from low to high style-hold and curl-retention properties, for example by varying the weight ratio between both latex polymers, with or without additives.

## Description of Exemplary Embodiments

The disclosure relates to a hair styling composition comprising at least two latex polymers chosen from acrylate and polyurethane polymers and optionally at least one component chosen from coalescing agents, plasticizers, and/or thickening agents, wherein at least one latex polymer is a film-forming polymer;
wherein the composition comprises at least one latex polymer A and at least one latex polymer B chosen from:

(a) polymer A, having a Young's modulus ranging from 0.1 MPa to 10 MPa and a strain, under stress at 0.5 MPa, of at least 1%; and

(b) polymer B, having a Young's modulus ranging from 10 MPa to 6 GPa and a strain, under stress at 0.5 MPa, of less than 5%;

wherein the at least two latex polymers are, independently or together, dispersed particles in an aqueous dispersion medium;
with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers; wherein the at least two latex polymers are present in a combined amount ranging from 0.1% to 8% by weight, relative to the weight of the composition; wherein the at least two latex polymers are present in the composition in a weight ratio ranging from 10: 1 to 1:10; and
wherein said composition produces a film having a Young's modulus ranging from 0.05 MPa to 5 GPa, and a strain, under stress at 0.5 MPa, that ranges up to 300%.

The composition comprising the at least two polymers forms a film when applied to a substrate. By way of example only, the film may have a Young's modulus ranging from 80 MPa to 5 GPa and a strain, under stress at 0.5 MPa, ranging from 0.01 % to less than 1%. By way of further example, the film may have a Young's modulus ranging from 5 MPa to 100 MPa and a strain, under stress at 0.5 MPa, ranging from 0.5% to less than 20%. By way of yet further example, the film may have a Young's modulus ranging from 0.05 MPa to 5 MPa and a strain, under stress at 0.5 MPa, ranging from 10% to 300%.

In at least certain exemplary embodiments according to the disclosure, the resulting film formed by the composition comprising at least two latex polymers, wherein at least one latex polymer is a film-forming polymer, is clear and/or transparent.

In further embodiments, methods of styling the hair are disclosed, said methods comprising applying compositions according to the disclosure to the hair. Such styling methods may comprise shaping, reshaping, positioning,

repositioning, adding volume to, curling, or straightening the hair, in order to achieve a certain hair style or appearance.

## LATEX POLYMERS

The at least two latex polymers, at least one of which is a film-forming polymer, are chosen from acrylate and polyurethane polymers, with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers.

The at least two latex polymers are present in a combined amount ranging from 0.1% to 8% by weight, relative to the weight of the composition, in a weight ratio of 10:1 to 1:10.

The at least two latex polymers may be identified as polymer A and polymer B. Compositions according to the present invention comprise at least one polymer A and at least one polymer B, wherein at least one of polymer A and polymer B is a film-forming polymer.

Polymer A is chosen from latex polymers having a Young's modulus ranging from 0.1 MPa to 10 MPa and a strain, under stress at 0.5 MPa, of at least 1%; and polymer B is chosen from latex polymers having a Young's modulus ranging from 10 MPa to 6 GPa and a strain, under stress at 0.5 MPa, of less than 5%. In at least certain embodiments, polymer A may have a glass transition temperature (Tg) ranging from -90°C to 40°C, and polymer B may have a glass transition temperature (Tg) ranging from 40°C to 200°C. In at least certain other embodiments, the weight ratio of polymer A to polymer B in the compositions of the disclosure is from 1:10 to 1:1, from 3:1 to 10:1, or from 5:1 to 10:1. Polymers A and B are chosen from acrylate and polyurethane polymers, with the proviso that when polymer A is chosen from an acrylate polymer, polymer B is chosen from a polyurethane polymer; and when polymer A is chosen from a polyurethane polymer, polymer B is chosen from an acrylate polymer.

In at least certain exemplary and non-limiting embodiments, latex polymers A and B may be chosen such that polymer A comprises at least one latex polymer which is optionally a film-forming polymer that is a relatively soft, flexible latex polymer, and polymer B comprises at least one latex polymer which is optionally a film-forming polymer that is a relatively hard, brittle polymer, although such characteristics are not required.

At least one of polymer A and polymer B is a film-forming polymer. In various exemplary embodiments, latex polymer A is a film-forming polymer and latex polymer B is a non-film-forming polymer. In further exemplary embodiments, latex polymer A is a non-film-forming polymer and latex polymer B is a film-forming polymer. In yet further exemplary embodiments, latex polymer A is a film-forming polymer and latex polymer B is a film-forming polymer.

As used herein, a film-forming polymer is meant to include a polymer that is capable, by itself or in the presence of an auxiliary film-forming agent, of forming a macroscopically continuous film that adheres to keratin materials, and preferably a cohesive film, better still, a film whose cohesion and mechanical properties are such that said film can be isolated and manipulated individually, for example, when said film is prepared by pouring onto a non-stick surface such as Teflon-coated or silicone-coated surface. In addition, as used herein, a non-film-forming polymer is meant to include a polymer which will not form a film at ambient temperature or below, or in other words, will only form a film at temperatures above ambient. For purposes of this disclosure, ambient temperature is taken as being below 40°C such as in the range of 15°C to 30°C.

By "at least two latex polymers," it is contemplated that more than two latex polymers may be chosen. Thus, for example, in various embodiments, both polymers A and B in the compositions of the disclosure may be latex film-forming polymers, and the composition may also comprise at least one latex polymer that is a non-film-forming polymer; or one of polymer A and B may be a film-forming polymer while the other is a non-film-forming polymer, but at least one additional film-forming (latex or non-latex) polymer may also be added; and so on. However, as described, the at least two latex polymers are chosen from acrylate and polyurethane polymers, with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers.

In further embodiments, the composition comprises exactly two latex polymers, at least one of which is a film-forming polymer. In yet further embodiments, the composition comprises at least two latex polymers, one or both of which are film-forming polymers, but does not comprise any additional film-forming polymers.

The at least two latex polymers are provided in the form of aqueous dispersions prior to formulating the compositions of the disclosure. In various embodiments, the aqueous dispersions may be obtained through an emulsion polymerization of monomers wherein the resulting latex polymers have a particle size lower than 1μm. In at least one exemplary embodiment, a dispersion prepared by the polymerization in water of one or more monomers having a polymerizable double bond may be chosen. In another exemplary embodiment, the aqueous dispersions obtained through an emulsion polymerization may be spray-dried.

In other embodiments, the latex polymers are produced from condensation reactions between monomers and subsequently dispersed in an aqueous medium.

Thus, the latex polymers exist as dispersed polymer particles in an aqueous dispersion medium. The latex

polymers may, in certain embodiments, each be dispersed in independent dispersion media. In yet further embodiments, the latex polymers may be dispersed together in the same dispersion medium.

The dispersion medium comprises at least one solvent chosen from water. The dispersion medium may further comprise at least one solvent chosen from cosmetically acceptable organic solvents. Cosmetically acceptable organic solvents may, in various embodiments, be water-miscible, e.g. capable of forming at 25°C a homogeneous mixture that is transparent, or substantially transparent, to the eye. For instance, cosmetically acceptable organic solvents may be chosen from lower monoalcohols, such as those containing from 1 to 5 carbon atoms, for example ethanol and isopropanol; polyols, including glycols, such as those containing from 2 to 8 carbon atoms, for example propylene glycol, ethylene glycol, 1,3-butylene glycol, dipropylene glycol, hexylene glycol, and glycerin; hydrocarbons, such as, for example, isododecane and mineral oil; and silicones, such as dimethicones, cyclomethicones, and cyclopentasiloxane; as well as mixtures thereof.

In at least one embodiment, the solvent of the dispersion medium consists of water. In other embodiments, the solvent of the dispersion medium consists of water and at least one cosmetically acceptable organic solvent. In further embodiments, the solvent comprises water. In yet further embodiments, the solvent of the dispersion medium primarily comprises water. For example, the solvent of the dispersion medium may, in at least certain exemplary embodiments, comprise greater than 50% water, such as greater than 55% water, greater than 60% water, greater than 65% water, greater than 70% water, greater than 75% water, greater than 80% water, greater than 85% water, greater than 90% water, greater than 95% water, greater than 96% water, greater than 97% water, greater than 98% water, or greater than 99% water.

In embodiments according to the disclosure, the latex polymer particles are not soluble in the solvent of the dispersion medium, i.e. are not water soluble and/or are not soluble in the at least one cosmetically acceptable organic solvent. Accordingly, the latex polymers retain their particulate form in the solvent or solvents chosen.

In at least certain exemplary embodiments, latex particles according to the disclosure may have an average diameter ranging up to 1000 nm, such as from 50 nm to 800 nm, or from 100 nm to 500 nm. Such particle sizes may be measured with a laser granulometer (e.g. Brookhaven BI90).

In various embodiments, the latex polymers may, independently, be neutralized, partially neutralized, or unneutralized. In exemplary embodiments where the latex polymers are neutralized or partially neutralized, the particle size may be, for example, greater than 800 nm. In at least certain embodiments, the particulate form of the latex polymers is retained in the dispersion medium.

In further embodiments, the latex polymers may be chosen from uncharged and charged latex polymers. Thus, the latex polymers may, according to various exemplary embodiments, be chosen from nonionic latex polymers, cationic latex polymers, and anionic latex polymers.

As non-limiting examples of latex polymers that may be used, mention may be made, independently, of acrylate latex polymers and polyurethane latex polymers. As described herein, it is to be understood that when the first latex polymer is chosen from an acrylate polymer, the second latex polymer is chosen from a polyurethane polymer; and when the first latex polymer is chosen from a polyurethane polymer, the second latex polymer is chosen from an acrylate polymer.

By way of non-limiting example only, one of the at least two latex polymers may be chosen from acrylate latex polymers, such as those resulting from the homopolymerization or copolymerization of monomers chosen from (meth)acrylics, (meth)acrylates, (meth)acrylamides and/or vinyl homopolymers or copolymers. The term "(meth)acryl" and variations thereof, as used herein, means acryl or methacryl.

The (meth)acrylic monomers may be chosen from, for example, acrylic acid, methacrylic acid, citraconic acid, itaconic acid, maleic acid, fumaric acid, crotonic acid, and maleic anhydride. Additional non-limiting examples of (meth)acrylic monomers include C1-C8 alkyl (meth)acrylic, such as, for example, methyl (meth)acrylic, ethyl (meth)acrylic, propyl (meth)acrylic, isopropyl (meth)acrylic, butyl (meth)acrylic, tert-butyl (meth)acrylic, pentyl(meth) acrylic, isopentyl (meth)acrylic, neopentyl (meth)acrylic, hexyl (meth)acrylic, isohexyl (meth)acrylic, 2-ethylhexyl (meth)acrylic, cyclohexyl (meth)acrylic, isohexyl (meth)acrylic, heptyl (meth)acrylic, isoheptyl (meth)acrylic, octyl (meth)acrylic, isooctyl (meth)acrylic, as well as combinations of any of the above.

The esters of (meth)acrylic monomers may be, by way of non-limiting example, C1-C8 alkyl (meth)acrylates such as methyl (meth)acrylate, ethyl (meth)acrylate, propyl (meth)acrylate, isopropyl (meth)acrylate, butyl (meth)acrylate, tert-butyl (meth)acrylate, pentyl(meth) acrylate, isopentyl (meth)acrylate, neopentyl (meth)acrylate, hexyl (meth)acrylate, isohexyl (meth)acrylate, 2-ethylhexyl (meth)acrylate, cyclohexyl (meth)acrylate, isohexyl (meth)acrylate, heptyl (meth)acrylate, isoheptyl (meth)acrylate, octyl (meth)acrylate, isooctyl (meth)acrylate, allyl (meth)acrylate, and combinations thereof. Additional and non-limiting examples include C1-C8 alkoxy (meth)acrylates, such as methoxy (meth)acrylate, ethoxy (meth)acrylate, propyl oxide (meth)acrylate, isopropyl oxide (meth)acrylate, butyl oxide (meth)acrylate, tert-butyl oxide (meth)acrylate, pentyl oxide (meth) acrylate, isopentyl oxide (meth)acrylate, neopentyl oxide (meth)acrylate. The esters may be, by way of non-limiting example, C2-C6 hydroxy alkyl (meth)acrylates, such as hydroxy ethyl (meth)acrylate, 2-hydroxypropyl (meth)acrylate, glycidyl (meth)acrylate, ethylene glycol di(meth)acrylate, polyethylene

glycol mono(meth)acrylate, 1,4-butane diol di(meth)acrylate, 1,6,hexane diol di(meth)acrylate, and any combination thereof. The esters may be, by way of non-limiting example, aryl (meth)acrylates such as benzyl (meth)acrylate, phenyl (meth)acrylate, and any combination thereof. The esters can further contain amino groups such as aminoethyl (meth)acrylate, N,N-dimethylaminoethyl (meth)acrylate, N,N-dimethylaminopropyl (meth)acrylate, N,N-dimethylaminodimethylpropyl (meth)acrylate, N,N-diethyleaminoethyl (meth)acrylate, and N,N,N-trimethylaminoethyl (meth)acrylate; and salts of the ethylenic amines.

According to at least certain exemplary embodiments, the alkyl group of the esters may be either fluorinated or perfluorinated, e.g. some or all of the hydrogen atoms of the alkyl group are substituted with fluorine atoms. The monomers can also be fluorine-containing monomers, such as, by way of non-limiting example, trifluoroethyl methacrylate, 2,2,3,3-tetrafluoropropyl methacrylate, 2,2,3,3,4,4-hexafluorobutyl methacrylate, perfluorooctyl methacrylate and perfluorooctyl acrylate; and silicone macromonomers.

The amides of (meth)acrylic monomers can, for example, be made of (meth)acrylamides, and especially N-alkyl (meth)acrylamides, in particular N-(C1-C12) alkyl (meth)acrylates such as N-ethyl (meth)acrylamide, N-t-butyl (meth)acrylamide, N-t-octyl (meth)acrylamide, N-methylol (meth)acrylamide and N-diacetone (meth)acrylamide, and any combination thereof.

The vinyl monomers can include, but are not limited to, vinyl cyanide compounds such as acrylonitrile and methacrylonitrile; vinyl esters such as vinyl formate, vinyl acetate, vinyl propionate, vinyl neodecanoate, vinyl pivalate, vinyl benzoate and vinyl t-butyl benzoate, triallyl cyanurate; vinyl halides such as vinyl chloride and vinylidene chloride; aromatic mono- or divinyl compounds such as styrene, $\alpha$-methylstyrene, chlorostyrene, alkylstyrene, divinylbenzene and diallyl phthalate, and combination thereof. Other non-limiting ionic monomers can include para-styrensulfonic, vinylsulfonic, 2-(meth)acryloyloxyethylsulfonic, 2-(meth)acrylamido-2-methylpropylsulfonic acids.

The list of monomers given is not limiting, and it should be understood that it is possible to use any monomer known to those skilled in the art which includes acrylic and/or vinyl monomers (including monomers modified with a silicone chain).

Silicone acrylic polymers may also optionally be used as vinyl polymer in at least one exemplary and non-limiting embodiment.

In at least certain, non-limiting exemplary embodiments, acrylic latex polymers may be chosen from aqueous dispersions of Methacrylic Acid/Ethyl Acrylate copolymer (INCI: Acrylates Copolymer, such as Luviflex® Soft by BASF), PEG/PPG-23/6 Dimethicone Citraconate/C10-30 Alkyl PEG-25 Methacrylate/Acrylic Acid/Methacrylic Acid/Ethyl Acrylate/Trimethylolpropane PEG-15 Triacrylate copolymer (INCI : Polyacrylate-2 Crosspolymer, such as Fixate Superhold ™ by Lubrizol), Styrene/Acrylic copolymer (such as Neocryl® A-1120, DSM), Ethylhexyl Acrylate/Methyl Methacrylate/Butyl Acrylate/Acrylic Acid/ Methacrylic Acid copolymer (INCI : Acrylates/Ethylhexyl Acrylate Copolymer, such as Daitosol 5000SJ, Daito Kasei Kogyo), Acrylic/Acrylates Copolymer (INCI name: Acrylates Copolymer, such as Daitosol 5000AD, Daito Kasei Kogyo), and Acrylic copolymers and Acrylates Copolymers, such as those known under the tradenames VINYSOL 2140 (Daido Chemical), ACULYN™ 33 (Dow Chemical), LUVIMER® MAE (BASF), or BALANCE CR (AKZO NOBEL).

In yet further exemplary and non-limiting embodiments, one of the at least two latex polymers may be chosen from polyurethane latex polymers, such as aqueous polyurethane dispersions comprising the reaction products of (i), (ii), and/or (iii), defined below.

Reaction product (i) may be any prepolymer according to the formula:

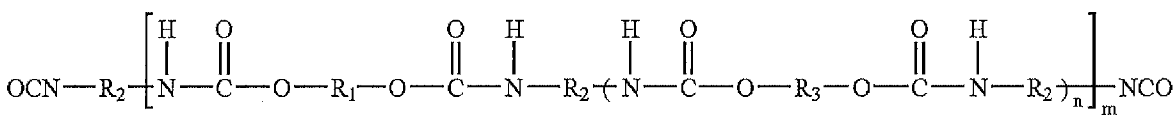

wherein R1 is chosen from bivalent radicals of a dihydroxyl functional compound, R2 is chosen from hydrocarbon radicals of an aliphatic or cycloaliphatic polyisocyanate, and R3 is chosen from radicals of a low molecular weight diol, optionally substituted with ionic groups, n ranges from 0 to 5, and m is greater than 1.

Suitable dihydroxyl compounds for providing the bivalent radical R1 include those having at least two hydroxy groups, and having number average molecular weights ranging from 700 to 16,000, such as, for example, from 750 to 5000. Non-limiting examples of the high molecular weight compounds include polyester polyols, polyether polyols, polyhydroxy polycarbonates, polyhydroxy polyacetals, polyhydroxy polyacrylates, polyhydroxy polyester amides, polyhydroxy polyalkadienes and polyhydroxy polythioethers. In various embodiments, polyester polyols, polyether polyols, and polyhydroxy polycarbonates may be chosen. Mixtures of such compounds are also within the scope of the disclosure.

The polyester diol(s) may optionally be prepared from aliphatic, cycloaliphatic, or aromatic dicarboxylic or polycarboxylic acids, or anhydrides thereof; and dihydric alcohols such as diols chosen from aliphatic, alicyclic, or aromatic diols.

The aliphatic dicarboxylic or polycarboxylic acids may be chosen from, for example: succinic, fumaric, glutaric, 2,2-dimethylglutaric, adipic, itaconic, pimelic, suberic, azelaic, sebacic, maleic, malonic, 2,2-dimethylmalonic, nonanedicarboxylic, decanedicarboxylic, dodecanedioic, 1,3-cyclohexanedicarboxylic, 1,4-cyclohexanedicarboxylic, 2,5-norboranedicarboxylic, diglycolic, thiodipropionic, 2,5-naphthalenedicarboxylic, 2,6-naphthalenedicarboxylic, phthalic, terephthalic, isophthalic, oxanic, o-phthalic, tetrahydrophthalic, hexahydrophthalic or trimellitic acid.

The acid anhydrides may, in further exemplary embodiments, be chosen from o-phthalic, trimellitic or succinic acid anhydride or a mixture thereof. By way of non-limiting example only, the dicarboxylic acid may be adipic acid.

The dihydric alcohols may be chosen from, for example, ethanediol, ethylene glycol, diethylene glycol, triethylene glycol, trimethylene glycol, tetraethylene glycol, 1,2-propanediol, dipropylene glycol, tripropylene glycol, tetrapropylene glycol, 1,3-propanediol, 1,4-butanediol, 1,3-butanediol, 2,3-butanediol, 1,5-pentanediol, 1,6-hexanediol, 2,2-dimethyl-1,3-propanediol, 1,4-dihydroxycyclohexane, 1,4-dimethylolcyclohexane, cyclohexanedimethanol, 1,8-octanediol, 1,10-decanediol, 1,12-dodecanediol, neopentyl glycol, and mixtures thereof. The cycloaliphatic and/or aromatic dihydroxyl compounds may also be suitable as the dihydric alcohol(s) for the preparation of the polyester polyol(s).

The polyester diols may also be chosen from homopolymers or copolymers of lactones, which are, in at least certain embodiments, obtained by addition reactions of lactones or lactone mixtures, such as butyrolactone, ε-caprolactone and/or methyl-ε-caprolactone with the appropriate polyfunctional, e.g. difunctional, starter molecules such as, for example, the dihydric alcohols mentioned above. The corresponding polymers of ε-caprolactone may be chosen in at least some embodiments.

The polyester polyol, e.g. polyester diol, radical R1, may be obtained by polycondensation of dicarboxylic acids, such as adipic acid, with polyols, e.g. diols, such as hexanediol, neopentyl glycol, and mixtures thereof.

The polycarbonates containing hydroxyl groups comprise those known per se, such as the products obtained by reacting diols, such as (1,3)-propanediol, (1,4)-butanediol and/or (1,6)-hexanediol, diethylene glycol, triethylene glycol, or tetraethylene glycol with diaryl carbonates, for example diphenyl carbonate or phosgene.

Optional polyether polyols may be obtained in any known manner by reacting starting compounds which contain reactive hydrogen atoms with alkylene oxides, such as, for example, ethylene oxide; propylene oxide; butylene oxide; styrene oxide; tetrahydrofuran; or epichlorohydrin, or with mixtures of these alkylene oxides. In at least certain embodiments, the polyethers do not contain more than 10% by weight of ethylene oxide units. For example, polyethers obtained without addition of ethylene oxide may be chosen.

Polyethers modified with vinyl polymers are also suitable according to various embodiments of the disclosure. Products of this type can be obtained by polymerization, for example, of styrene and acrylonitrile in the presence of polyethers, for example as described in U.S. Patent Nos. 3,383,351; 3,304,273; 3,523,095; 3,110,695; and German patent 1 152 536.

Among the polythioethers which may be chosen include the condensation products obtained from thiodiglycol per se and/or with other glycols, dicarboxylic acids, formaldehyde, aminocarboxylic acids, and/or amino alcohols. The products obtained are either mixed polythioethers, polythioether esters, or polythioether ester amides, depending on the co-components.

Optional polyacetals include but are not limited to the compounds which can be prepared from aldehydes, for example formaldehyde, and from glycols, such as diethylene glycol, triethylene glycol, ethoxylated 4,4'-(dihydroxy)diphenyl-dimethylmethane, and (1,6)-hexanediol. Polyacetals useful according to various non-limiting embodiments of the disclosure can also be prepared by polymerization of cyclic acetals.

Optional polyhydroxy polyesteramides and polyamines include, for example, the mainly linear condensation products obtained from saturated or unsaturated, polybasic carboxylic acids or anhydrides thereof, and from saturated or unsaturated, polyvalent amino alcohols, from diamines, or from polyamines, as well as mixtures thereof.

Optional monomers for the production of polyacrylates having hydroxyl functionality comprise acrylic acid, methacrylic acid, crotonic acid, maleic anhydride, 2-hydroxyethyl acrylate, 2-hydroxyethyl methacrylate, 2-hydroxypropyl acrylate, 2-hydroxypropyl methacrylate, 3-hydroxypropyl acrylate, 3-hydroxypropyl methacrylate, glycidyl acrylate, glycidyl methacrylate, 2-isocyanatoethyl acrylate, and 2-isocyanatoethyl methacrylate.

Mixtures of dihydroxy compounds can also be chosen.

Optional polyisocyanates for providing the hydrocarbon-based radical R2 include, for example, organic diisocyanates having a molecular weight ranging from 100 to 1500, such as 112 to 1000, or 140 to 400.

Optional diisocyanates are those chosen from the general formula $R_2(NCO)_2$, in which $R_2$ represents a divalent aliphatic hydrocarbon group comprising from 4 to 18 carbon atoms, a divalent cycloaliphatic hydrocarbon group comprising from 5 to 15 carbon atoms, a divalent araliphatic hydrocarbon group comprising from 7 to 15 carbon atoms, or a divalent aromatic hydrocarbon group comprising from 6 to 15 carbon atoms. Examples of the organic diisocyanates which may be chosen include, but are not limited to, tetramethylene diisocyanate, 1,6-hexamethylene diisocyanate, dodecamethylene diisocyanate, cyclohexane-1,3-diisocyanate and cyclohexane-1,4-diisocyanate, 1-isocyanato-3-isocyanatomethyl-3,5,5-trimethylcyclohexane (isophorone diisocyanate or IPDI), bis(4-isocyanatocyclohexyl)-methane, 1,3-bis(isocyanatomethyl)cyclohexane and 1,4-bis(isocyanatomethyl)cyclohexane and bis(4-isocyanato-3-methylcyclohexyl)methane. Mixtures of diisocyanates can also be used.

In at least certain embodiments, diisocyanates are chosen from aliphatic and cycloaliphatic diisocyanates. For example, 1,6-hexamethylene diisocyanate, isophorone diisocyanate, and dicyclohexylmethane diisocyanate, as well as mixtures thereof may be chosen.

The use of diols, for example low molecular weight diols, R3, may in at least certain embodiments allow a stiffening of the polymer chain. The expression "low molecular weight diols" means diols having a molecular weight ranging from 50 to 800, such as 60 to 700, or 62 to 200. They may, in various embodiments, contain aliphatic, alicyclic, or aromatic groups. In certain exemplary embodiments, the compounds contain only aliphatic groups. The diols that may be chosen may optionally have up to 20 carbon atoms, and may be chosen, for example, from ethylene glycol, diethylene glycol, propane-1,2-diol, propane-1,3-diol, butane-1,4-diol, 1,3-butylene glycol, neopentyl glycol, butylethylpropanediol, cyclohexanediol, 1,4-cyclohexanedimethanol, hexane-1,6-diol, bisphenol A (2,2-bis(4-hydroxyphenyl)propane), hydrogenated bisphenol A (2,2-bis(4-hydroxycyclohexyl)propane), and mixtures thereof. For example, R3 may be derived from neopentyl glycol.

Optionally, the low molecular weight diols may contain ionic or potentially ionic groups. Suitable low molecular weight diols containing ionic or potentially ionic groups may be chosen from those disclosed in U.S. Patent No. 3,412,054. In various embodiments, compounds may be chosen from dimethylolbutanoic acid (DMBA), dimethylolpropionic acid (DMPA), and carboxyl-containing caprolactone polyester diol. If low molecular weight diols containing ionic or potentially ionic groups are chosen, they may, for example, be used in an amount such that less than 0.30 meq of -COOH is present per gram of polyurethane in the polyurethane dispersion. In at least certain exemplary and non-limiting embodiments, the low molecular weight diols containing ionic or potentially ionic groups are not used.

Reaction product (ii) may be chosen from at least one chain extender according to the formula:

$$H2N - R4 - NH2$$

wherein R4 is chosen from alkylene or alkylene oxide radicals, said radicals not being substituted with ionic or potentially ionic groups.

Reaction product (ii) may optionally be chosen from alkylene diamines, such as hydrazine, ethylenediamine, propylenediamine, 1,4-butylenediamine and piperazine; and alkylene oxide diamines such as dipropylamine diethylene glycol (DPA-DEG available from Tomah Products, Milton, WI), 2-methyl-1,5-pentanediamine (Dytec A from DuPont), hexanediamine, isophoronediamine, and 4,4-methylenedi(cyclohexylamine), and the DPA-series of ether amines available from Tomah Products, Milton, WI, including dipropylamine propylene glycol, dipropylamine dipropylene glycol, dipropylamine tripropylene glycol, dipropylamine poly(propylene glycol), dipropylamine ethylene glycol, dipropylamine poly(ethylene glycol), dipropylamine 1,3-propanediol, dipropylamine 2-methyl-1,3-propanediol, dipropylamine 1,4-butanediol, dipropylamine 1,3-butanediol, dipropylamine 1,6-hexanediol and dipropylamine cyclohexane-1,4-dimethanol, and mixtures thereof.

Reaction product (iii) may be chosen from at least one chain extender according to the formula:

$$H2N-R5-NH2$$

wherein R5 is chosen from alkylene radicals substituted with ionic or potentially ionic groups. In at least certain exemplary embodiments, the compounds may have an ionic or potentially ionic group and two isocyanate-reactive groups.

As used herein, ionic or potentially ionic groups may include groups comprising ternary or quaternary ammonium groups, groups convertible into such groups, carboxyl groups, carboxylate groups, sulphonic acid groups, and sulphonate groups. At least partial conversion of the groups convertible into salt groups of the type mentioned may take place before or during the mixing with water. Specific compounds include diaminosulphonates, such as for example the sodium salt of N-(2-aminoethyl)-2-aminoethanesulphonic acid (AAS) or the sodium salt of N-(2-aminoethyl)-2- aminopropionic acid.

In at least certain embodiments, R5 represents an alkylene radical substituted with sulphonic acid or sulphonate groups. By way of example only, the compound is chosen from sodium salts of N-(2-aminoethyl)-2-aminoethanesulphonic acid.

By way of non-limiting example, such latexes include, but are not limited to, aqueous polyurethane dispersions comprising a reaction product of a prepolymer comprising a dihydroxyl compound, a polyisocyanate, and a low molecular weight diol and at least two diamine compounds and wherein the composition is substantially free of triethanolamine stearate such as, for example, those sold under the BAYCUSAN® name by Bayer such as, for example, BAYCUSAN® C1000 (INCI name: Polyurethane-34), BAYCUSAN® C1001 (INCI name: Polyurethane-34), BAYCUSAN® C1003 (INCI name: Polyurethane-32), BAYCUSAN® C1004 (INCI name: Polyurethane-35) and BAYCUSAN® C1008 (INCI name: Polyurethane-48). In various exemplary embodiments, polyurethane latexes may be chosen from, but are not limited to, aqueous polyurethane dispersion of Isophthalic Acid/Adipic Acid/Hexylene Glycol/Neopentyl glycol/Dimethylolpropanoic Acid/Isophorone Diisocyanate copolymer (INCI name: Polyurethane-1, such as Luviset® P.U.R, BASF), aliphatic

polyurethane and aliphatic polyester polyurethane (such as the Neorez® series, DSM, such as Neorez® R989, INCI name: Polycarbamyl Polyglycon Ester).

In at least certain embodiments, the at least two latex polymers may be chosen from polyacrylic latex, polyacrylate latex, polystyrene latex, polyester latex, polyamide latex, polyurea latex, polyurethane latex, epoxy resin latex, cellulose-acrylate latex, and their copolymers.

In various embodiments according to the disclosure, it may be possible to choose a polymer that comprises both acrylate and polyurethane parts at the molecular level.

## COALESCING AGENTS AND PLASTICIZERS

The compositions according to the present invention optionally comprise at least one component chosen from coalescing agents and plasticizers. Without wishing to be bound by theory, it is believed that the addition of coalescing agents and/or plasticizers may lower the glass transition temperature (Tg), decrease the Young's modulus, and increase the strain of latex polymers and/or the films formed by latex polymers. Further, the at least one coalescing agent and/or plasticizer may also be used to aid coating formation of the latex film to form a continuous and homogeneous film or coating and to improve adhesion. While the lowering of the Tg of the latex polymers can result in a softening of the film or coating formed by the latex polymers, it has been found that the coating or film produced on hair treated with the compositions of the disclosure surprisingly and unexpectedly imparts a strong styling hold to the hair while leaving the hair with a natural/clean feel and look. As such, the flexibility and stiffness of the resulting film or coating may be more balanced, and thus impart a better style and stronger hold to hair.

Non-limiting examples of coalescing agents and/or plasticizers that may be used according to various embodiments include glycols and their derivatives, such as glycol ethers, for example, ethylene glycol, propylene glycol, diethylene glycol ethyl ether, diethylene glycol methyl ether, diethylene glycol butyl ether, diethylene glycol hexyl ether, diethylene glycol dibutyl ether, ethylene glycol methyl ether, ethylene glycol ethyl ether, ethylene glycol butyl ether, and ethylene glycol hexyl ether; glycol esters, such as diethylene glycol butyl ether acetate, propylene glycol dibenzoate and dipropylene glycol dibenzoate; cellulose esters, such as sucrose acetate; propylene glycol derivatives, such as propylene glycol phenyl ether, propylene glycol diacetate, dipropylene glycol butyl ether, tripropylene glycol butyl ether, propylene glycol methyl ether, dipropylene glycol ethyl ether, tripropylene glycol methyl ether and diethylene glycol methyl ether, and propylene glycol butyl ether.

According to further embodiments, acid esters, for example carboxylic acid esters, may be chosen. Non-limiting examples include acetates, such as glycerol triacetate; citrates, such as triethyl citrate, tributyl citrate, triethyl acetylcitrate, tributyl acetylcitrate and tri(2-ethylhexyl)acetylcitrate; phthalates, such as diethyl phthalate, dibutyl phthalate, dioctyl phthalate, dipentyl phthalate, dimethoxyethyl phthalate, butyl phthalate, and 2-ethylhexyl phthalate; phosphates, such as tricresyl phosphate, tributyl phosphate, triphenyl phosphate and tributoxyethyl phosphate; tartrates, such as dibutyl tartrate; and sebacates, such as dimethyl sebacate and dibutyl sebacate.

Furthermore, fatty acid esters, such as adipic acid esters, may be chosen. Non-limiting examples include diisobutyl adipate and diethyl adipate. Stearic acid esters, such as ethyl stearate, and palmitic acid esters, such as 2-ethylhexyl palmitate, succinates, abietates, caprylates, caproates, enanthates, and myristates may also be chosen.

In further embodiments, the at least one component chosen from coalescing agents and plasticizers may be chosen from carbonates, such as ethylene carbonate and propylene carbonate; benzyl benzoate, sucrose benzoate, butyl acetylricinoleate, glyceryl acetylricinoleate, butyl glycolate, camphor, N-ethyl-o,p-toluenesulphonamide, and ethyl tosylamide.

In yet further embodiments, oxyethylenated derivatives, such as oxyethylenated oils, may be chosen, for example,vegetable oil, castor oil, oils of natural origin, including non-drying oils and those comprising at least one fatty acid chosen from caprylic acid, capric acid, lauric acid, myristic acid, palmitic acid, stearic acid, oleic acid, ricinoleic acid, linoleic acid, linolenic acid, arachidic acid, gadoleic acid, behenic acid, erucic acid, brassidic acid, cetoleic acid, lignoceric acid and nervonic acid. In at least certain exemplary embodiments, the oils are chosen from triglycerides composed of esters of fatty acids and of glycerol, of which the fatty acids have varied chain lengths from C4 to C24 that can be linear or branched and saturated or unsaturated. Non-limiting examples of oils include heptanoic or octanoic triglycerides, groundnut, babassu, coconut, grape seed, cottonseed, maize, maize germ, mustard seed, palm, rapeseed, sesame, soybean, sunflower, wheat germ, canola, apricot, mango, castor, shea, avocado, olive, sweet almond, almond, peach, walnut, hazelnut, macadamia, jojoba, alfalfa, poppy, pumpkinseed, cucumber, blackcurrant, evening primrose, millet, barley, guinea, rye, safflower, candlenut, passionflower, musk rose or shea butter oils or triglycerides of caprylic/capric acids. In yet further exemplary embodiments, the oils may be chosen from alcohols such as hexanol and benzyl alcohol.

In preferred embodiments, the coalescing agents and/or plasticizers that may be used in the compositions of the disclosure include propylene glycol dibenzoate, available under the tradename, Lexfeel® Shine from the company Inolex, dipropylene glycol dibenzoate, available under the tradename, DERMOL DPG-2b from the company Alzo, and propylene glycol butyl ether, available under the tradename, DOWANOL™ PnB from the company Dow Chemical.

It should be understood that mixtures of the above agents may be used according to various embodiments.

THICKENING AGENTS/RHEOLOGY MODIFIERS

The compositions according to various embodiments of the disclosure also optionally comprise at least one component chosen from thickening agents, also referred to interchangeably herein as thickeners or rheology modifiers. Thickening agents are generally used to modify the viscosity or rheology of compositions. However, without wishing to be bound by theory, it is believed that the presence of thickening agents in compositions according to the disclosure may lower the glass transition temperature, Tg, decrease the Young's modulus, and increase the strain of latex polymers and/or the films formed by latex polymers. In addition, without wishing to be bound by theory, it is believed that the addition of the at least one thickening agent may aid in the distribution of the composition on hair, may ease handling and/or manageability of the composition. Thus, while thickening agents may decrease the Tg of the film formed by the composition, thereby softening the film or coating formed by the latex polymers, it was surprisingly and unexpectedly found that the coating or film produced on hair treated with the compositions of the disclosure imparts a strong styling hold to the hair while leaving the hair with a natural/clean feel and look. As such, the flexibility and stiffness of the resulting film or coating may be more balanced and thus impart a better style and stronger hold to hair. It is also possible to render the hair softer, and/or generally improve the performance of the composition on the hair.

Non-limiting examples of thickening agents that may be used according to various embodiments of the disclosure include those conventionally used in cosmetics, such as polymers of natural origin and synthetic polymers. For example, nonionic, anionic, cationic, amphiphilic, and amphoteric polymers, and other known rheology modifiers, such as cellulose-based thickeners, may be chosen.

The thickening agents may be chosen from, for example, hydrophilic thickeners, for example cellulose polymers and gums. As used herein, the term "hydrophilic thickener" is meant to indicate that the thickening agent is soluble or dispersible in water. Non-limiting examples of hydrophilic thickeners include modified or unmodified carboxyvinyl polymers, such as the products sold under the name CARBOPOL (CTFA name: carbomer) by Goodrich, homopolymers or copolymers of acrylic or methacrylic acids or the salts thereof and the esters thereof, such as the products sold under the names VERSICOL F® or VERSICOL K® by Allied Colloid, ULTRAHOLD 8® by Ciba-Geigy, polyacrylates and polymethacrylates such as the products sold under the names LUBRAJEL and NORGEL by Guardian, or under the name HISPAJEL by Hispano Chimica, and polyacrylic acids of SYNTHALEN K type, polyacrylamides, copolymers of acrylic acid and of acrylamide sold in the form of the sodium salt thereof, such as under the names RETEN® by Hercules, the sodium polymethacrylate such as sold under the name DARVAN 7® by Vanderbilt, and the sodium salts of polyhydroxycarboxylic acids such as sold under the name HYDAGEN F® by Henkel, optionally crosslinked and/or neutralized 2-acrylamido-2-methylpropanesulphonic acid polymers and copolymers, for instance poly(2-acrylamido-2-methylpropanesulphonic acid) such as sold by Clariant under the name HOSTACERIN AMPS (CTFA name: ammonium polyacryldimethyltauramide), crosslinked anionic copolymers of acrylamide and of AMPS, e.g. in the form of a water-in-oil emulsion, such as those sold under the name SEPIGEL™ 305 (CTFA name: Polyacrylamide/C13-14 Isoparaffin/Laureth-7) and under the name SIMULGEL™ 600 (CTFA name: Acrylamide/Sodium acryloyldimethyltaurate copolymer/Isohexadecane/Polysorbate 80) by SEPPIC, polyacrylic acid/alkyl acrylate copolymers of PEMULEN type, associative polymers, for instance PEG-150/stearyl alcohol/SMDI copolymer such as sold under the name ACULYN™ 46 by Rohm & Haas, steareth-100/PEG-136/HDI copolymer such as sold under the name RHEOLATE® FX 1100 by Elementis), as well as mixtures thereof.

Other exemplary hydrophilic thickeners include associative polymers. As used herein, the term "associative polymer" is intended to mean any amphiphilic polymer comprising in its structure at least one fatty chain and at least one hydrophilic portion. The associative polymers in accordance various exemplary embodiments may be anionic, cationic, nonionic or amphoteric. By way of example, associative polymers which may be chosen include those comprising at least one hydrophilic unit and at least one fatty-chain allyl ether unit, such as those in which the hydrophilic unit is constituted of an ethylenic unsaturated anionic monomer, such as a vinylcarboxylic acid or an acrylic acid, a methacrylic acid, and mixtures thereof, and in which the fatty-chain allyl ether unit corresponds to the monomer of formula (I) below:

$$CH_2 = C(R')CH_2OB_nR \qquad (I)$$

in which R' is chosen from H or $CH_3$, B is chosen from an ethyleneoxy radical, n is zero or is chosen from an integer ranging from 1 to 100, and R is chosen from a hydrocarbon-based radical chosen from alkyl, arylalkyl, aryl, alkylaryl and cycloalkyl radicals containing from 8 to 30 carbon atoms, such as from 10 to 24 carbon atoms, or from 12 to 18 carbon atoms. Exemplary and non-limiting polymers of this type are described and prepared, according to an emulsion polymerization process, in patent EP 0 216 479.

Non-limiting examples of associative anionic polymers that may also be chosen include anionic polymers comprising at least one hydrophilic unit of olefinic unsaturated carboxylic acid type, and at least one hydrophobic unit

exclusively of ($C_{10}$-$C_{30}$)alkyl ester of unsaturated carboxylic acid type. Examples that may be mentioned include, but are not limited to, the anionic polymers described and prepared according to patents U.S. Patent Nos. 3,915,921 and 4,509,949.

Cationic associative polymers that may be chosen include, but are not limited to, quaternized cellulose derivatives and polyacrylates containing amine side groups.

Exemplary non-ionic associative polymers include celluloses modified with groups comprising at least one fatty chain, for instance hydroxyethyl celluloses modified with groups comprising at least one fatty chain, such as alkyl groups, e.g. $C_8$-$C_{22}$ alkyl groups, arylalkyl and alkylaryl groups, such as cetyl hydroxyethyl cellulose, also known as Natrosol® Plus (sold by the company Ashland); Bermocoll EHM 100 (sold by the company Berol Nobel), Amercell Polymer HM-1500® sold by Amerchol (hydroxyethylcellulose modified with a polyethylene glycol (15) nonylphenyl ether group, sold by the company Amerchol), celluloses modified with polyalkylene glycol alkylphenyl ether groups, guars such as hydroxypropyl guar, optionally modified with groups comprising at least one fatty chain such as an alkyl chain, for example JAGUAR® XC-95/3 (C14 alkyl chain, sold by the company Rhodia Chimie); Esaflor HM 22 (C22 alkyl chain, sold by the company Lamberti); RE210-18 (C14 alkyl chain) and RE205-1 (C20 alkyl chain, sold by the company Rhodia Chimie), copolymers of vinylpyrrolidone and of fatty-chain hydrophobic monomers, for instance Antaron® or Ganex® V216 (vinylpyrrolidone/hexadecene copolymers); Antaron® or Ganex® V220 (vinylpyrrolidone/eicosene copolymers), sold by the company I.S.P., copolymers of $C_1$-$C_6$ alkyl methacrylates or acrylates and of amphiphilic monomers comprising at least one fatty chain, and copolymers of hydrophilic methacrylates or acrylates and of hydrophobic monomers comprising at least one fatty chain, for instance the polyethylene glycol methacrylate/lauryl methacrylate copolymer.

Associative polyurethanes may also be chosen in various exemplary and non-limiting embodiments. These are nonionic block copolymers comprising in the chain both hydrophilic blocks usually of polyoxyethylene nature, and hydrophobic blocks that may be aliphatic sequences alone and/or cycloaliphatic and/or aromatic sequences. Associative polyurethanes comprise at least two hydrocarbon-based lipophilic chains containing from $C_6$ to $C_{30}$ carbon atoms, separated by a hydrophilic block, the hydrocarbon-based chains optionally being pendent chains or chains at the end of a hydrophilic block. For example, it is possible for one or more pendent chains to be provided. In addition, the polymer may comprise a hydrocarbon-based chain at one or both ends of a hydrophilic block. The associative polyurethanes may be arranged in triblock or multiblock form. The hydrophobic blocks may thus be at the each end of the chain (for example, triblock copolymer with a hydrophilic central block) or distributed both at the ends and within the chain (for example, multiblock copolymer). These polymers may also be graft polymers or starburst polymers. For example, the associative polyurethanes may be triblock copolymers in which the hydrophilic block is a polyoxyethylene chain containing from 50 to 1000 oxyethylene groups.

By way of non-limiting example, associative polymers of the polyurethane polyether type that may be used include the polymer $C_{16}$-$OE_{120}$-$C_{16}$ from Servo Delden (under the name SER AD FX1100), which is a molecule containing a urethane function and having a weight-average molecular weight of 1300), OE being an oxyethylene unit, Nuvis® FX 1100 (European and US INCI name "Steareth-100/PEG-136/HMDI Copolymer" sold by the company Elementis Specialties), and also Acrysol RM 184® (sold by the company Rohm and Haas); Elfacos® T210® (C12-C14 alkyl chain) and Elfacos® T212® (C18 alkyl chain) sold by the company Akzo. Further exemplary associative polymers that may be chosen include RHEOLATE® 205 containing a urea function, sold by Rheox, or RHEOLATE® 208 or 204, or RHEOLATE® FX1100 from Elementis. The product DW 1206B from Rohm & Haas containing a $C_{20}$ alkyl chain with a urethane bond, sold at a solids content of 20% in water, may also be used.

In yet further exemplary embodiments, solutions or dispersions of these polymers, especially in water or in aqueous-alcoholic medium, may be chosen. Examples of such polymers include SER AD FX1010, SER AD FX1035 and SER AD 1070 from Servo Delden, and RHEOLATE® 255, RHEOLATE® 278 and RHEOLATE® 244 sold by Rheox. Further examples include the products ACULYN™ 46, DW 1206F and DW 1206J, and also ACRYSOL RM 184 or ACRYSOL 44 from Rohm & Haas, and BORCHIGEL LW 44 from Borchers.

In at least one exemplary embodiment, the at least one thickening agent is chosen from copolymers resulting from the polymerization of at least one monomer (a) chosen from carboxylic acids possessing $\alpha,\beta$-ethylenically unsaturated groups or their esters, with at least one monomer (b) possessing ethylenically unsaturated groups and comprising a hydrophobic group. Such copolymers may exhibit emulsifying properties.

As used herein, the term "copolymers" is intended to mean both copolymers obtained from two types of monomers and those obtained from more than two types of monomers, such as, for example, terpolymers obtained from three types of monomers. The chemical structure of the copolymers comprises at least one hydrophilic unit and at least one hydrophobic unit. The expression "hydrophobic unit" or "hydrophobic unit" is understood to mean a radical possessing a saturated or unsaturated and linear or branched hydrocarbon-based chain which comprises at least 8 carbon atoms, for example from 10 to 30 carbon atoms, as a further example from 12 to 30 carbon atoms, and as yet a further example from 18 to 30 carbon atoms.

In certain exemplary and non-limiting embodiments, the thickening copolymers are chosen from the copolymers resulting from the polymerization of:

(1) at least one monomer of formula (II):

CH2=CH(R1)COOH (II)

wherein $R_1$ is chosen from H or $CH_3$ or $C_2H_5$, providing acrylic acid, methacrylic acid, or ethacrylic acid monomers, and (2) at least one monomer of ($C_{10}$-$C_{30}$)alkyl ester of unsaturated carboxylic acid type corresponding to the monomer of formula (III):

CH2=CH(R2)COOR3 (III)

wherein $R_2$ is chosen from H or $CH_3$ or $C_2H_5$, providing acrylate, methacrylate or ethacrylate units, $R_3$ denoting a $C_{10}$-$C_{30}$ alkyl radical, such as a $C_{12}$-$C_{22}$ alkyl radical.

Non-limiting examples of ($C_{10}$-$C_{30}$)alkyl esters of unsaturated carboxylic acids are for example chosen from lauryl acrylate, stearyl acrylate, decyl acrylate, isodecyl acrylate, dodecyl acrylate and the corresponding methacrylates, such as lauryl methacrylate, stearyl methacrylate, decyl methacrylate, isodecyl methacrylate and dodecyl methacrylate, and mixtures thereof.

Additionally, crosslinked thickening polymers may be chosen according to further exemplary embodiments. For example, such polymers may be chosen from polymers resulting from the polymerization of a mixture of monomers comprising:

(1) acrylic acid,
(2) an ester of formula (III) described above, in which $R_2$ is chosen from H or $CH_3$, $R_3$ denoting an alkyl radical having from 12 to 22 carbon atoms, and
(3) a crosslinking agent, which is a well-known copolymerizable polyethylenic unsaturated monomer, such as diallyl phthalate, allyl (meth)acrylate, divinylbenzene, (poly)ethylene glycol dimethacrylate and methylenebisacrylamide.

By way of example, crosslinked thickening polymers comprising 60% to 95% by weight of acrylic acid (hydrophilic unit), 4% to 40% by weight of $C_{10}$-$C_{30}$ alkyl acrylate (hydrophobic unit), and 0% to 6% by weight of crosslinking polymerizable monomer. In yet further embodiments, the crosslinked thickening polymers may comprise 96% to 98% by weight of acrylic acid (hydrophilic unit), 1% to 4% by weight of $C_{10}$-$C_{30}$ alkyl acrylate (hydrophobic unit), and 0.1% to 0.6% by weight of crosslinking polymerizable monomer, such as those described above.

For example, acrylate/$C_{10}$-$C_{30}$ alkyl acrylate copolymers (INCI name: Acrylates/C10-30 Alkyl Acrylate Crosspolymer), such as the products sold by Lubrizol under the trade names PEMULEN™ TR1, PEMULEN™ TR2, CARBOPOL® 1382 and CARBOPOL® EDT 2020 may be chosen.

In further embodiments, the at least one thickening agent may be chosen from nonionic homopolymers or copolymers containing ethylenically unsaturated monomers of the ester and/or amide type. For example, the products sold under the names CYANAMER P250 by the company CYTEC (polyacrylamide), methyl methacrylate/ethylene glycol dimethacrylate copolymers (such as PMMA MBX-8C by the company US COSMETICS), butyl methacrylate/methyl methacrylate copolymers (such as ACRYLOID B66 by the company RHOM HMS), and polymethyl methacrylates (BPA 500 by the company KOBO) may be chosen.

In yet further embodiments, the at least one thickening agent chosen from polymers of natural origin may include, for example, thickening polymers comprising at least one sugar unit, for instance nonionic guar gums, optionally modified with C1-C6 hydroxyalkyl groups; biopolysaccharide gums of microbial origin, such as scleroglucan gum (also known as sclerotium gum) or xanthan gum; gums derived from plant exudates, such as gum arabic, ghatti gum, karaya gum, gum tragacanth, carrageenan gum, agar gum and carob gum, ceratonia siliqua gum and cyamopsis tetragonoloba (guar) gum; pectins; alginates; starches; hydroxy(C1-C6)alkylcelluloses and carboxy(C1-C6)alkylcelluloses.

Non-limiting examples of nonionic, unmodified guar gums that may be used in various embodiments include Guargel D/15 (Noveon); Vidogum GH 175 (Unipectine), Meypro-Guar 50 and JAGUAR® C (Meyhall/Rhodia Chimie). Non-limiting examples of nonionic modified guar gums include Jaguar® HP8, HP60, HP120, DC 293 and HP 105 (Meyhall/Rhodia Chimie); and Galactasol 4H4FD2 (Ashland).

Further examples of useful thickening agents include scleroglucans, for example, Actigum™ CS from Sanofi Bio Industries; Amigel from Alban Muller International, and also the glyoxal-treated scleroglucans described in FR2633940); xanthan gums, for instance Keltrol®, Keltrol® T, Keltrol® Tf, Keltrol® Bt, Keltrol® Rd, Keltrol® Cg (Nutrasweet Kelco), Rhodicare® S and Rhodicare® H (Rhodia Chimie); starch derivatives, for instance Primogel® (Avebe); hydroxyethylcelluloses such as Cellosize® QP3L, QP4400H, QP30000H, HEC30000A and Polymer PCG10 (Amerchol), Natrosol™ 250HHR®, 250MR, 250M, 250HHXR, 250HHX, 250HR, HX (Hercules) and Tylose® H1000 (Hoechst); hydroxypropylcelluloses, for instance Klucel® EF, H, LHF, MF and G (Ashland); carboxymethylcelluloses, for instance

Blanose® 7M8/SF, refined 7M, 7LF, 7MF, 9M31F, 12M31XP, 12M31P, 9M31XF, 7H, 7M31, 7H3SXF (Ashland), Aquasorb® A500 (Hercules), Ambergum® 1221 (Hercules), Cellogen® HP810A, HP6HS9 (Montello) and Primellose® (Avebe).

Exemplary modified nonionic guar gums may, for example, be modified with C1-C6 hydroxyalkyl groups. Exemplary hydroxyalkyl groups may include hydroxymethyl, hydroxyethyl, hydroxypropyl and hydroxybutyl groups.

Guar gums are well known in the state of the art and may, for example, be prepared by reacting the corresponding alkene oxides, such as for example propylene oxides, with guar gum so as to obtain a guar gum modified with hydroxypropyl groups. The hydroxyalkylation ratio, which corresponds to the number of alkylene oxide molecules consumed to the number of free hydroxyl functional groups present on the guar gum, may in at least certain exemplary embodiments vary from about 0.4 to 1.2.

Exemplary and non-limiting nonionic guar gums, optionally modified with hydroxyalkyl groups, include those sold under the trade names JAGUAR® HP8, JAGUAR® HP60 and JAGUAR® HP120, JAGUAR® DC 293 and JAGUAR® HP 105 by the company RHODIA CHIMIE (RHODIA CHIMIE), and under the name GALACTASOL™ 4H4FD2 by the company ASHLAND.

Guar gums may also be modified with a quaternary ammonium group. Guar gums modified as such include Guar Hydroxypropyltrimonium Chloride, also known under the tradename JAGUAR® C-13S (RHODIA CHIMIE).

Exemplary and non-limiting celluloses include hydroxyethylcelluloses and hydroxypropylcelluloses. The products sold under the names KLUCEL EF, KLUCEL H, KLUCEL LHF, KLUCEL MF, KLUCEL G, by the company ASHLAND, CELLOSIZE POLYMER PCG-10 by the company AMERCHOL, may be chosen in various embodiments.

Exemplary, non-limiting thickening polysaccharides may be chosen from glucans, modified or unmodified starches (such as those derived, for example, from cereals such as wheat, corn or rice, vegetables such as golden pea, tubers such as potato or cassava), amylose, amylopectin, glycogen, dextrans, celluloses and derivatives thereof (methylcelluloses, hydroxyalkylcelluloses, ethylhydroxyethylcelluloses), mannans, xylans, lignins, arabans, galactans, galacturonans, chitin, chitosans, glucoronoxylans, arabinoxylans, xyloglucans, glucomannans, pectic acids and pectins, arabinogalactans, carrageenans, agars, gums arabic, gums tragacanth, Ghatti gums, Karaya gums, carob gums, galactomannans such as guar gums and their nonionic derivatives (hydroxypropylguar), and mixtures thereof.

Further, exemplary thickening agents include silicas, optionally hydrophobic, such as those described in EP-A-898960, and for example marketed as AEROSIL® R812 by the company Degussa, CAB-O-SIL TS-530, CAB-O-SIL TS-610, CAB-O-SIL TS-720 by the company Cabot, AEROSIL® R972, AEROSIL® R974 by the company Degussa; clays, such as montmorillonite, modified clays such as the bentones for example, stearalkonium hectorite, stearalkonium bentonite; polysaccharide alkyl ethers (optionally with the alkyl group having from 1 to 24 carbon atoms, for example from 1 to 10 carbon atoms, as a further example from 1 to 6 carbon atoms, and as yet a further example from 1 to 3 carbon atoms) such as those described in document EP-A-898958.

Thickening agents of the present disclosure may also include rheology modifiers. In accordance with the disclosure, rheology modifiers may, in various exemplary embodiments, be chosen from Polyacrylamide(and)C13-14 Isoparaffin(and)Laureth-7 (Sepigel™ 305 from Seppic), Hydroxypropyl Guar (JAGUAR® HP105 from Rhodia), Cyamopsis Tetragonoloba (Guar) Gum (Supercol U Guar Gum from Ashland), Acrylates/C10-30 Alkyl Acrylate Crosspolymer (Carbopol® Ultrez 20 Polymer from Lubrizol), Acrylates/C10-30 Alkyl Acrylate Crosspolymer (Permulen™ TR-1 from Lubrizol), Polyacrylate Crosspolymer-6 (Sepimax Zen from Seppic), Sclerotium Gum (Amigum from Alban Muller), Xanthan Gum(and)Ceratonia Siliqua Gum (Nomcort CG from Nisshin Oil Lio), Hydroxypropyl Guar (Jaguar® HP8 from Rhodia), Guar Hydroxypropyl Trimonium Chloride (Jaguar® C-13-S from Rhodia), Hydroxyethyl Cellulose (Natrosol® 250 MR from Ashland).

When anionic thickening agents are used, they are generally neutralized before being included in or as they are added to the compositions of the disclosure. Such anionic thickening agents may be neutralized by employing traditional neutralizing agents such as alkanolamines, for example, monoethanolamine and diethanolamine; aminomethyl propanol; basic amino acids, for example arginine and lysine; and ammonium compounds and their salts. The anionic thickening agents may also be neutralized by at least one latex polyurethane polymer of the disclosure wherein said latex polyurethane polymer has at least one free amino group and/or is provided in a dispersion medium that has a pH of greater than 7.

Cationic thickening agents of the disclosure may also be chosen from non-associative cationic polymers such as dimethylaminoethyl methacrylate homopolymers quaternized with methyl chloride or dimethylaminoethyl methacrylate copolymers quaternized with methyl chloride and acrylamide. Among the homopolymers of this type, mention may be made of the products sold under the names Salcare SC95 and Salcare SC96 by the company Ciba and SYNTHALEN® CR by the company 3V Sigma (chemical name: methacryloylethyl trimethyl ammonium chloride homopolymer, INCI name: polyquaternium-37). Among the copolymers of this family, mention may be made of the product Salcare S C92 sold by Ciba or the product PAS 5 194 sold by Hoechst.

Another suitable example of a cationic thickening agent is a product known by the INCI name of polyacrylate-1 crosspolymer (Carbopol® Aqua CC, from the company, Lubrizol).

It is contemplated that, in at least certain exemplary and non-limiting embodiments, the thickening agents of the disclosure may include compounds such as gellifying and viscosity modifying agents. For example, compositions of the disclosure may employ at least one water-soluble resin such as polyethylene oxide having a molecular weight ranging from 100,000 to 10,000,000. Examples of such polyethylene oxides include, but not limited to, Polyox water-soluble resins manufactured by Dow under the INCI names of PEG-2M, PEG-5M, PEG-7M, PEG-14M, PEG-23M, PEG-45M, PEG-90M, PEG-160M, and PEG-180M. PEG-90M is known under the tradename of Polyox™ WSR 301, and PEG-45M is known under the tradename Polyox™ WSR 60k. The amounts of water-soluble resins in the compositions, when present, may range from 0.1% to 2% by weight relative to the total weight of the composition.

It is to be understood that any combination of the above mentioned agents is contemplated according to various exemplary embodiments of the disclosure.

**COMPOSITIONS**

As described herein, compositions according to the disclosure comprise at least two latex polymers chosen from acrylate and polyurethane polymers, wherein at least one of the latex polymers is a film-forming polymer, with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers.

In certain embodiments, each of the latex polymers is present in an amount ranging from 0.1% to 7.5% by weight, such as 0.25% to 5% by weight, such as 0.5% to 2.5% by weight, or 0.5% to 1.5% by weight, relative to the weight of the composition, including all ranges and subranges there between. In other embodiments, each of the latex polymers is present in an amount ranging from 0.1 % to 2% by weight, such as 0.15% to 1.9% by weight, or such as 0.18% to 1.8% by weight, relative to the weight of the composition, including all ranges and subranges there between.

In certain embodiments, the latex polymers are present in a combined amount ranging from 0.2% to 5% by weight, such as 0.5% to 5% by weight, such as 1% to 5% by weight, such as 1% to 3% by weight, relative to the weight of the composition, including all ranges and subranges there between. By way of non-limiting example, the combined amount of latex polymers may be 0.1%, 0.5%, 1%, 2%, 3%, 4%, 5%, 6%, 7%, 8%, by weight, relative to the weight of the composition.

In yet further embodiments, the combined amount of latex polymers ranges up to 8%, up to 7%, up to 6%, up to 5%, up to 4%, up to 3%, up to 2%, or up to 1%, each by weight, relative to the weight of the composition. In at least one exemplary embodiment, the combined amount of latex polymers is less than than 5% by weight, relative to the weight of the composition.

According to various embodiments of the disclosure, the weight ratio of the at least two latex polymers, e.g. polymer A to polymer B, may range from 9:1 to 1:9, 8:1 to 1:8, 7:1 to 1:7, 6:1 to 1:6, 5:1 to 1:5, 4:1 to 1:4, 3:1 to 1:3, or 2:1 to 1:2, including all ranges and subranges there between. It should be understood that when polymer A and/or polymer B comprise at least one latex film-forming polymer, the weight ratio includes the total amount of polymer A and/or polymer B.

According to various embodiments of the disclosure, the weight ratio of polymer A to polymer B is 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10.

Different weight ratios of polymer A to polymer B may be chosen to correspond to different hair styling applications. By way of example only, a weight ratio of polymer A to polymer B ranging from 1:10 to 1:1 may, in some embodiments, provide a high level of style hold; a weight ratio of polymer A to polymer B ranging from 5:1 to 10:1 may, in some embodiments, provide a medium to high level of style hold; and a weight ratio of polymer A to polymer B ranging from 3:1 to 10:1 may, in some embodiments, provide a light to medium level of style hold.

According to various embodiments, the at least one component chosen from coalescing agents and plasticizers may be present in an amount ranging from 0.1% to 20% by weight, such as from 0.1% to 10% by weight, or from 0.1% to 5% by weight, with respect to the total weight of the composition. In at least one exemplary embodiment, the at least one component chosen from coalescing agents and plasticizers ranges from 0.1% to 2% by weight, and in a further exemplary embodiment from 0.1% to 1% by weight, with respect to the total weight of the composition.

According to various embodiments, the at least one component chosen from thickening agents may be present in an amount ranging from 0.1% to 10% by weight, such as from 0.1% to 5% by weight, or from 0.5% to 4% by weight, or from 1% to 3% by weight, based on the total weight of the composition.

In addition to the at least two latex polymers, wherein at least one is a film-forming polymer, and optional thickening agents and/or at least one component chosen from coalescing agents and plasticizers, the compositions may further comprise at least one solvent. The at least one solvent may be chosen from water, at least one cosmetically acceptable organic solvent, or a mixture of water and at least one cosmetically acceptable organic solvent. Cosmetically acceptable organic solvents may, in various embodiments, be water-miscible, e.g. a mixture capable of forming at 25°C a homogeneous mixture that is transparent, or substantially transparent, to the eye. For instance, cosmetically acceptable

organic solvents may be chosen from lower monoalcohols, such as those containing from 1 to 5 carbon atoms, for example ethanol and isopropanol; polyols, including glycols, such as those containing from 2 to 8 carbon atoms, for example propylene glycol, ethylene glycol, 1,3-butylene glycol, dipropylene glycol, hexylene glycol, and glycerin; hydrocarbons, such as, for example, isododecane and mineral oil; and silicones, such as dimethicones, cyclomethicones, and cyclopentasiloxane; as well as mixtures thereof.

The at least one solvent may be present in an amount ranging up to 95%, such as from 1% to 90%, from 5% to 80%, or from 10% to 60% by weight, relative to the total weight of the composition.

In at least certain exemplary embodiments, the latex polymer particles are not soluble in the solvent of the composition, and thus remain in particulate form even after evaporation of the solvent. For example, in embodiments where the composition comprises alcohol as a cosmetically acceptable organic solvent, the latex particles may remain in particulate form upon evaporation of the alcohol, such as once the composition is applied to a substrate.

Compositions according to various embodiments of the disclosure may further comprise additional components that are typically used in hair styling compositions. Such components are known to those of skill in the art, or are within the ability of those of skill in the art to determine depending on the particular application, such as, for example, the particular component and/or amount thereof.

In various embodiments, the composition described herein may have a pH ranging from 2 to 9, such as 3 to 8, or 4 to 7.

In at least certain exemplary embodiments, the compositions are in the form of hair styling compositions, in any form, such as, for example, a gel, a cream, a foam, a lotion, an emulsion, or a liquid that may be sprayed onto or otherwise applied to the hair. In various embodiments, the composition may be provided in the form of a gel, a mousse, or a spray. In at least certain embodiments, the composition may be applied to the hair by first applying to the hands, and then contacting the hair with the hands; in other embodiments, the composition may be applied directly onto the hair, such as by spraying. The compositions may, in various embodiments, be applied to the hair as a leave-on treatment.

In various embodiments, the application of an external stimuli, such as heat, may be desirable as part of the hair styling process. By way of example only, before, during, or after the composition is applied to wet or dry hair, the hair may optionally be further treated with an external stimuli, for example with heat ranging from 25°C to 250°C. In at least certain embodiments, the hair may also be shaped or positioned as desired while exposed to external stimuli, such as while heated or exposed to heat.

Professional and consumer heating tools can be used as a means to deliver heat or an elevated temperature to the hair. The heating tools can generate heat through electrical current or heating lamps. Depending upon the desired style, these tools include, but are not limited to, heaters, blow dryers, flat irons, hot combs, hot curler sets, steam pods, heated crimpers, heated lash curlers, heated wands/brushes, and hood driers or their combinations thereof.

As described, compositions according to the disclosure may impart a film on a substrate, such as on the hair or on the hand during or after application to the hair. A film formed by the composition may, surprisingly, be clean-feeling and not sticky, as with traditional hair styling compositions. Also surprisingly, the composition may impart a film on the hair that leaves the hair relatively natural and clean-feeling, yet has a flexible coating, leaving little to no residue, allows the hair to be bouncy and springy with little to no frizz or flaking, may impart relatively high definition with individualized curls, style control, volume, and shine, and/or may allow for relatively long-lasting hold and style memory. Furthermore, in at least certain embodiments according to the disclosure, the compositions are not sticky or tacky. A user of hair compositions according to various embodiments described herein may thus feel that the composition is not perceptible or is "invisible," yet still effectively style and/or hold the hair. Additionally, the compositions may have effective hair styling and/or hold properties, even in conditions of high, or relatively high, humidity. In at least certain embodiments according to the disclosure, the compositions may be quick-drying, which may allow drying and/or styling time to be reduced, as well as further improve ease of styling and curl retention.

Furthermore, as described, compositions prepared according to various embodiments may provide for varying degrees of hold to be imparted to a hair style. By way of non-limiting example only, in order to obtain a spiky look to hair of a very short length, a high level of styling hold may be desirable. Or, as a further non-limiting example, in order to obtain a flowing look or to maintain hair curls for hair of medium length or longer length, a light to medium level of style hold may be desirable. By altering the weight ratio of the first and second polymers, it is possible to formulate compositions having high levels of style hold, medium to high levels of style hold, medium levels of style hold, or light to medium levels of style hold.

In at least certain embodiments, a film formed by the compositions described herein may be clear and/or stable. In such embodiments, phase separation and dramatic aggregation are minimized.

In addition, hair styled or treated with compositions according to the disclosure may, in at least certain exemplary embodiments, be hydrophobic, and/or may appear less frizzy and/or may be less prone to breakage, relative to hair subjected to the same conditions but not having been styled or treated with a composition according to the disclosure.

It should be noted, however, that compositions and films, as well as hair to which the composition or film has been applied, according to the disclosure may not have one or more of the herein-referenced properties, yet are intended

to be within the scope of the disclosure.

Also disclosed herein are methods for styling the hair, said methods comprising applying a composition according to the disclosure to the hair, either before, during, or after styling the hair. One or more steps of treating the hair with an external stimuli, such as heat, before, during, or after the composition has been applied to the hair are also contemplated.

It is to be understood that both the foregoing description and the following Examples are exemplary and explanatory only, and are not to be interpreted as restrictive of the disclosure. Moreover, it should be understood that various features and/or characteristics of differing embodiments herein may be combined with one another. It is therefore to be understood that numerous modifications may be made to the illustrative embodiments and that other arrangements may be devised without departing from the scope of the disclosure. Other embodiments will be apparent to those skilled in the art from consideration of the disclosure and practice of the various exemplary embodiments disclosed herein.

It is also to be understood that, as used herein the terms "the," "a," or "an," mean "at least one," and should not be limited to "only one" unless explicitly indicated to the contrary. Thus, for example, the use of "a surfactant" is intended to mean at least one surfactant.

Unless otherwise indicated, all numbers used in the specification and claims are to be understood as being modified in all instances by the term "about," whether or not so stated. The term "about" as it modifies numbers herein is meant to indicate a difference of 10% or less from the stated number, such as 9% or less, such as 8% or less, such as 7% or less, such as 6% or less, such as 5% or less, such as 4% or less, such as 3% or less, such as 2% or less, or such as 1% or less, in various exemplary embodiments. Thus, by way of example only, in one embodiment where "about" indicates a difference of 10% or less, the phrase "about 20%" is intended to encompass a range from 18%-22%. In another exemplary embodiment where "about" indicates a difference of 5% or less, the phrase "about 20%" is intended to encompass a range from 19%-21%. All such numbers within each specified range are hereby explicitly intended to be included in the disclosure.

It should also be understood that the precise numerical values used in the specification and claims form additional embodiments of the disclosure, and are intended to include any ranges which can be narrowed to any two end points disclosed within the exemplary ranges and values provided, as well as the specific end points themselves. Efforts have been made to ensure the accuracy of the numerical values disclosed herein. Any measured numerical value, however, can inherently contain certain errors resulting from the standard deviation found in its respective measuring technique.

It should be understood that compositions according to various embodiments of the disclosure form a film when applied to a substrate. However, the various properties of the film described herein are intended to include any film provided by compositions according to the disclosure, regardless of whether the film is attached or bonded to the substrate or not. By way of example only, once the compositions are applied to a substrate and a film is formed, the film may subsequently be removed in order to evaluate properties such as strain and Young's modulus.

## EXAMPLES

The following Examples are intended to be non-restrictive and explanatory only, with the scope of the invention being defined by the claims.

### Procedures

A. Procedures for Determination of Physical Properties of Films

Film plating: The latex film was obtained by allowing a 30 gram water solution containing 4 grams of the latex polymer(s) to dry slowly in a 100 mL PFA Petri dish (100 mm diameter x 15 mm height) at room temperature for at least 3 days.

Film measurement: The latex film, with known dimensions (length, width, thickness), was mounted on the Q800 Dynamic Mechanical Analysis from TA Instrument, and tested in a DMA Control Force mode. The stress/strain test was obtained using the following procedure:

Preload force: 0.001 N

Isothermal: 25°C

Soak time: 0.5 minutes

Force ramp rate: 0.5 MPa/min to 18 N

The test ended when the sample broke, 18 N force was reached, or maximum displacement was achieved (25.5 mm).

From the stress/strain curve, the Young's Modulus was calculated as the slope of the linear portion at 0.01% Strain to 1% Strain. From the stress/strain curve, the %Strain at the stress of 0.5 MPa was also reported.

A high Young's Modulus demonstrates a hard film, while a lower Young's Modulus represents a more elastic film. A high Strain demonstrates a stretchy, elastic film, while a lower Strain represents a more brittle film.

B. Procedure for Determination of Mechanical Properties of Hair Treated With Latex Compositions

Hair treatment: A strip of regular bleached hair (from IHIP, 1 cm in width, 16 cm long, about 2.0 - 2.5 g of hair) was treated with the latex solution (0.75 g of solution/g hair). The hair was combed through until the solution was uniformly distributed over the surface of the tress. The treated hair was allowed to dry overnight at room temperature.

Hair measurement: Three-point bending measurements were conducted using a texture analyzer (Model TA-XTPlus, Texture Technologies Corporation) equipped with a hair mounting accessory as described in J. Cosmet. Sci., 53, 345-362 (November/December 2002). The cantilever bending experiment consisted of the following sequence of steps: the hair tress was placed on a 2-point of 6 cm width, and the probe, representing the third point, came down at the middle of the hair tress and performed 10 cycles of 10-mm deformations of the hair tress. The testing protocol was:

Test mode = Compression

Pre-test speed = 2 mm/sec

Test speed = 2 mm/sec

Post-test speed = 2 mm/sec

Target mode = Distance

Distance = 10 mm

Count = 10

Trigger type = Auto (Force)

Trigger force = 1 g

After finishing 10 cycles of bending, a plot of force as a function of distance of 10 deformations was generated. From the plot, the maximum force in the first (F1) and the tenth (F10) deformation cycle was determined. The change from F1 to F10 was calculated from:

$$(F1-F10) / F1 \times 100.$$

A high maximum force indicated that the hair was stiff and rigid, and a lower maximum force indicated that the hair was softer and more flexible.

Each experiment was run three times, and the results are reported from the average of the three experiments.

C. Procedure for Determination of Curl Retention in High Humidity of Hair Treated With Latex Compositions

Hair treatment: Regular bleached hair swatch (from IHIP, 14.5 cm long, 0.5 g) was treated with a solution of 2% latex polymers (0.5g solution/g hair). The hair was combed until the solution was uniformly distributed over the hair swatch surface. The treated hair was then rolled onto a spiral rod (0.5 in diameter) and allowed to dry at room temperature overnight.

Curl retention measurement: The coiled hair was removed from the rod and placed in the humidity chamber at 95% RH, 25°C for 24 hours. The Curl Retention was calculated as:

$$(Lo-Lf) / (Lo-Li) \times 100$$

wherein Lo = fully extended hair length, Li = initial coiled hair length before humidity exposure, and Lf = final hair length after 24 hours exposure

Compositions containing latex polymers were evaluated according to the methods described above. The weight of each latex polymer in the following examples is determined on a dry weight basis.

Example 1: Evaluation of Acrylate Latex-Polyurethane Latex Combinations

Clear films were obtained from the combination of DAITOSOL 5000AD (INCI name: Acrylates Copolymer, Young's Modulus of 0.4 MPa and strain, under stress at 0.5 MPa, of >150%; polymer A) and NEOREZ® R989 (INCI name: Polycarbamyl Polyglycon Ester, Young's Modulus of 654 MPa and strain, under stress at 0.5 MPa, of 0.07%; polymer B) at various latex polymer ratios. Their physical properties are shown in Table 1.

**TABLE 1**

| Sample | Component (A:B) | Young's Modulus (MPa) | Strain at 0.5 MPa stress (%) |
|---|---|---|---|
| 1a | Polymer A only | 0.4 | >150 |
| 1b | 1:10 | 429 | 0.09 |
| 1c | 1:5 | 354 | 0.14 |
| 1d | 1:3 | 274 | 0.24 |
| 1e | 1:1 | 86 | 0.61 |
| 1f | 3:1 | 17 | 5.97 |
| 19 | 5:1 | 3 | 130.20 |
| 1h | 10:1 | 0.5 | >130 |
| 1i | Polymer B only | 654 | 0.07 |

These results show that by varying the ratio of the two latex polymers, it is possible to control the hardness (not as hard as Polymer A and not as soft as Polymer B) and flexibility (not as brittle as Polymer A and not as stretchy as Polymer B) of films produced according to various embodiments of the disclosure.

Example 2: Evaluation of Polyurethane Latex-Acrylate Latex Combinations

Clear films were obtained from the combination of BAYCUSAN® C1001 (INCI name: Polyurethane-34, Young's Modulus of 3 MPa and strain, under stress at 0.5 MPa, of 18.82%; polymer A) and LUVIFLEX® SOFT (INCI name: Acrylates copolymer, Young's Modulus of 2758 MPa and strain, under stress at 0.5 MPa, of <0.01 %; polymer B) at various latex polymer ratios. Their physical properties are shown in Table 2 below.

**TABLE 2**

| Sample | Component (A:B) | Young's Modulus (MPa) | Strain at 0.5 MPa stress (%) |
|---|---|---|---|
| 2a | Polymer A only | 3 | 18.82 |
| 2b | 1:10 | 2476 | 0.02 |
| 2c | 1:5 | 1617 | 0.03 |
| 2d | 1:3 | 1609 | 0.02 |
| 2e | 1:1 | 506 | 0.07 |
| 2f | 3:1 | 28 | 0.77 |
| 2g | 5:1 | 22 | 1.76 |
| 2h | 10:1 | 11 | 5.89 |
| 2i | Polymer B only | 2758 | <0.01 |

These results show that by varying the ratio of the two latex polymers, it is possible to control the hardness

(not as hard as Polymer A and not as soft as Polymer B) and flexibility (not as brittle as Polymer A and not as stretchy as Polymer B) of films produced according to various embodiments of the disclosure.

Example 3: Evaluation of Hair Treated with Acrylate Latex-Polyurethane Latex Combinations

Hair tresses were treated with 2% solutions of DAITOSOL 5000AD (polymer A) and NEOREZ® R989 (polymer B) at various latex polymer ratios. Their mechanical properties are shown in Table 3 below.

**TABLE 3**

| Sample | Component (A:8) | F1 (g) | Change in F (%) |
|---|---|---|---|
| 3a | Polymer A only | 124 | 35 |
| 3b | 1:10 | 930 | 46 |
| 3c | 1:5 | 705 | 18 |
| 3d | 1:3 | 791 | 41 |
| 3e | 1:1 | 588 | 25 |
| 3f | 3:1 | 332 | 44 |
| 3g | 5:1 | 280 | 34 |
| 3h | 10:1 | 188 | 36 |
| 3i | Polymer B only | 1257 | 30 |
| 3j | Commercial 1* | 1835 | 76 |
| *Main ingredients: VP/VA copolymer, polyquaternium-11, PEG 90 M, PEG-40 hydrogenated castor oil, acrylates/C10-30 alkyl acrylate crosspolymer, alcohol denatured. | | | |

These results show that hair tresses treated with various ratios of the two latex polymers display a wide variety of rigidity, flexibility, stiffness, and softness. Compared to a commercial product (no latex), they show a significantly better styling durability due to the lower change in the maximum force after 10 cycles of deformation.

Example 4: Evaluation of Hair Treated with Polyurethane Latex-Acrylate Latex Combinations

Hair tresses were treated with 2% solutions of BAYCUSAN® C1001 (polymer A) and LUVIFLEX® SOFT (polymer B) at various latex polymer ratios. Their mechanical properties are shown in Table 4 below.

**TABLE 4**

| Sample | Component (A:B) | F1 (g) | Change in F (%) |
|---|---|---|---|
| 4a | Polymer A only | 399 | 27 |
| 4b | 1:10 | 1038 | 29 |
| 4c | 1:5 | 1091 | 27 |
| 4d | 1:3 | 1143 | 22 |
| 4e | 1:1 | 512 | 34 |
| 4f | 3:1 | 635 | 42 |
| 4g | 5:1 | 438 | 44 |
| 4h | 10:1 | 449 | 36 |
| 4i | Polymer B only | 945 | 29 |
| 4j | Commercial 1* | 1835 | 76 |
| * Main ingredients: VP/VA copolymer, polyquaternium-11, PEG 90 M, PEG-40 hydrogenated castor oil, acrylates/C10-30 alkyl acrylate crosspolymer, alcohol denatured. | | | |

These results show that hair tresses treated with various ratios of the two latex polymers display a wide variety of rigidity, flexibility, stiffness, and softness. Compared to a commercial product (no latex), they show a significantly better styling durability due to the lower change in the maximum force after 10 cycles of deformation.

Example 5: Evaluation of High Humidity Curl Retention of Hair Treated with Acrylate Latex-Polyurethane Latex Combinations

Hair swatches were treated with 2% solutions of DAITOSOL 5000AD (polymer A) and NEOREZ® R989 (polymer B) at various latex polymer ratios. The high humidity curl retention results are shown in Table 5 below.

**TABLE** 5

| Sample | Component (A:B) | Curl Retention (%) |
|---|---|---|
| 5a | Polymer A only | 32 |
| 5b | 1:10 | 90 |
| 5c | 1:5 | 84 |
| 5d | 1:3 | 76 |
| 5e | 1:1 | 63 |
| 5f | 3:1 | 46 |
| 5g | 5:1 | 33 |
| 5h | 10:1 | 32 |
| 5i | Polymer B only | 82 |

These results show that addition of the second latex improves the curl retention, compared to the individual latexes.

Example 6: Evaluation of High Humidity Curl Retention of Hair Treated with Polyurethane Latex-Acrylate Latex Combinations

Hair swatches were treated with 2% solutions of BAYCUSAN® C1001 (polymer A) and LUVIFLEX® SOFT (polymer B) at various latex polymer ratios. The high humidity curl retention results are shown in Table 6A below.

**TABLE 6A**

| Sample | Component (A:B) | Curl Retention (%) |
|---|---|---|
| 6a | Polymer A only | 40 |
| 6b | 1:10 | 77 |
| 6c | 1:5 | 79 |
| 6d | 1:3 | 71 |
| 6e | 1:1 | 55 |
| 6f | 3:1 | 80 |
| 6g | 5:1 | 66 |
| 6h | 10:1 | 55 |
| 6i | Polymer B only | 71 |

Hair swatches were treated with 2% solutions of BAYCUSAN® C1001 (polymer A) and ACULYN 33™ (INCI name: Acrylates Copolymer, Young's Modulus of 2096 MPa and strain, under stress at 0.5 MPa, of 0.01%; polymer B) at various latex polymer ratios. Their curl retention results are shown in Table 6B below.

**TABLE 6B**

| Sample | Component (A:B) | Curl Retention (%) |
|---|---|---|
| 6j | 1:3 | 76 |
| 6k | 1:2 | 82 |
| 6l | 1:1 | 64 |

Hair swatches were treated with 2% solutions of BAYCUSAN® C1001 (polymer A) and LUVIMER® MAE (INCI name: Acrylates copolymer, Young's Modulus of 385 MPa and strain, under stress at 0.5 MPa, of <1%; polymer B) and at various latex polymer ratios. Their curl retention results are shown in Table 6C below.

**TABLE 6C**

| Sample | Component (A:B) | Curl Retention (%) |
|---|---|---|
| 6m | 1:1 | 76 |
| 6m | 2:1 | 76 |
| 6o | 3:1 | 70 |

The results seen in Tables 6A, 6B, and 6C show that addition of the second latex improves the curl retention, compared to the individual latexes.

Example 7: Evaluation of Effects of Concentration on Performance on Treated Hair

Regular bleached hair was treated with solutions of 1:1 ratio of LUVIFLEX® SOFT and BAYCUSAN® C1001 at various latex polymer concentrations. The three-point bending test and the high humidity curl retention test was performed as described above. The results are shown in Table 7 below.

**TABLE 7**

| Sample | Concentration | F1 (g) | Curl Retention (%) |
|---|---|---|---|
| 7a | 1% | 263 | 58 |
| 7b | 2% | 499 | 69 |
| 7c | 5% | 1381 | 95 |
| 7d | None (commercial 1*) | 1835 | 42 |
| 7e | None (commercial 2**) | 4394 | 58 |
| *Main ingredients: VP/VA copolymer, polyquaternium-11, PEG 90 M, PEG-40 hydrogenated castor oil, acrylates/C10-30 alkyl acrylate crosspolymer, alcohol denatured.<br>**Main ingredients: Water, Acrylates/steareth-20 methacrylate crosspolymer, polyquaternium-69, PVP, sorbitol and alcohol denatured. | | | |

The results demonstrate that as the concentration of the latexes increases, the hardness of the styled hair increases, as well as an increase in curl retention. It is noted that while having a wide range of hold, styled hair shows a significantly better hydrophobicity and humidity resistance compared to that treated with commercial (no latex) products.

Example 8: Evaluation of Effects of Coalescing Agents on Hardening Rate

Hair tresses were treated with compositions comprising NEOREZ® R989 and DAITOSOL® 5000AD in a combined weight of 2% and at 1:1 latex polymer ratio, and 0%, 0.3% and 0.6% of DOWANOL™ PNB (INCI name: Propylene Glycol Butyl Ether (and) Propylene Glycol Butyl Ether (and) BHT). The film hardening rates are shown below in Table 8.

**TABLE 8**

| Sample | Coalescing Agent | Slope | $R^2$ | Range (g) | Increase in Force |
|---|---|---|---|---|---|
| 8a | 0% | 0.2720 | 0.99 | 11.77 - 23.16 | 97% |
| 8b | 0.3% | 0.3087 | 0.99 | 10.18 - 23.16 | 99% |
| 8c | 0.6% | 0.3452 | 0.97 | 10.26 - 20.48 | 127% |

These results show that as the amount of the coalescing agents in the latex solution increases, the film on the hair hardens at an increasing rate.

Example 9: Evaluation of Effects of Coalescing Agents on Hardening Rate

Hair tresses were treated with 2% solutions of LUVIFLEX® SOFT and BAYCUSAN® C1001 at 1:1 latex polymer ratio containing 0%, 0.3% and 0.6% of DOWANOL™ PNB. The film hardening rates are shown in Table 9 below.

**TABLE 9**

| Sample | Coalescing Agent | Slope (hardening rate) | $R^2$ | Range (g) | Increase in Force |
|---|---|---|---|---|---|
| 9a | 0% | 0.5108 | 0.99 | 8.85 - 27.49 | 210% |
| 9b | 0.3% | 0.7370 | 0.98 | 12.88 - 40.53 | 214% |
| 9c | 0.6% | 0.8843 | 0.98 | 10.01 - 42.52 | 324% |

These results show that as the amount of the coalescing agent in the latex solution increases, the film on the hair hardens at an increasing rate.

Example 10: Evaluation of High Humidity Curl Retention

Hair swatches were treated with 2% solutions of DAITOSOL 5000AD and NEOREZ® R989, or LUVIFLEX® SOFT and BAYCUSAN® C1001, at 1:1 latex polymer ratio containing 0%, 0.3% and 0.6% of DOWANOL™ PNB. The high humidity curl retention results are shown in Table 10 below.

**TABLE 10**

| Sample | Latex (1:1) | Coalescing Agent | Curl Retention |
|---|---|---|---|
| 10a | NEOREZ R989:DAITOSOL 5000AD | 0% | 72% |
| 10b | NEOREZ R989:DAITOSOL 5000AD | 0.3% | 72% |
| 10c | NEOREZ R989:DAITOSOL 5000AD | 0.6% | 73% |
| 10d | LUVIFLEX SOFT:BAYCUSAN C1001 | 0% | 86% |
| 10e | LUVIFLEX SOFT:BAYCUSAN C1001 | 0.3% | 87% |
| 10f | LUVIFLEX SOFT:BAYCUSAN C1001 | 0.6% | 87% |

These results show that addition of the coalescing agent improves the curl retention at high humidity at the rates shown, compared to compositions not comprising coalescing agents.

Example 11: Evaluation of Thickener in Acrylate-Polyurethane Latex Composition

Hair tresses were treated with a gel containing 1% Guar Gum (INCI name: Cyamopsis tetragonoloba (Guar) Gum) and 2% of NEOREZ® R989 and DAITOSOL 5000AD at 1:1 latex polymer ratio. The physical property of the hair is shown in Table 11A.

**TABLE 11A**

| Sample | F1 (g) |
|---|---|
| NO THICKENER | 588 |
| WITH THICKENER | 1163 |

Hair tresses were treated with a gel containing 1% Carbopol Ultrez-20 (INCI name: Acrylates/C10-C30 Alkyl Acrylate Crosspolymer) and 2% solutions of LUVIFLEX® SOFT and BAYCUSAN® C1001 at 1:1 latex polymer ratio. The physical property of the hair is shown in Table 11B.

**TABLE 11B**

| Sample | F1 (g) |
|---|---|
| NO THICKENER | 512 |
| WITH THICKENER | 1438 |

These results demonstrate that that addition of a thickener to the latex solution makes the hair stiffer, giving it more hold.

Example 12: Evaluation of High Humidity Curl Retention

Hair swatches were treated with a gel containing 1% Guar Gum and 2% NEOREZ® R989 and DAITOSOL 5000AD or a gel containing 1% Carbopol Ultrez-20 and 2% LUVIFLEX® SOFT and BAYCUSAN® C1001 at 1:1 latex polymer ratio. Their high humidity curl retention results are shown below in Table 12.

**TABLE 12**

| Sample | Curl Retention (%) |
|---|---|
| NEOREZ R989/DAITOSOL 5000AD, no thickener | 69 |
| NEOREZ R989/DAITOSOL 5000AD, with thickener | 91 |
| LUVIFLEX SOFT/BAYCUSAN C1001, no thickener | 84 |
| LUVIFLEX SOFT/BAYCUSAN C1001, with thickener | 90 |
| Commercial 1* | 57 |
| Commercial 2** | 63 |
| *Main ingredients: VP/VA copolymer, polyquaternium-11, PEG 90 M, PEG-40 hydrogenated castor oil, acrylates/C10-30 alkyl acrylate crosspolymer, alcohol denatured.<br>**Main ingredients: Water, Acrylates/steareth-20 methacrylate crosspolymer, polyquaternium-69, PVP, sorbitol and alcohol denatured. | |

These results demonstrate that addition of a thickener to the latex solution improves the curl retention of the hair.

## Claims

1. A hair styling composition comprising at least two latex polymers chosen from acrylate and polyurethane polymers and optionally at least one component chosen from coalescing agents, plasticizers, and/or thickening agents, wherein at least one latex polymer is a film-forming polymer;
   wherein the composition comprises at least one latex polymer A and at least one latex polymer B chosen from:

   (a) polymer A, having a Young's modulus ranging from 0.1 MPa to 10 MPa and a strain, under stress at 0.5 MPa, of at least 1%; and
   (b) polymer B, having a Young's modulus ranging from 10 MPa to 6 GPa and a strain, under stress at 0.5 MPa, of less than 5%;

wherein the at least two latex polymers are, independently or together, dispersed particles in an aqueous dispersion medium;
with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers;
wherein the at least two latex polymers are present in a combined amount ranging from 0.1% to 8% by weight, relative to the weight of the composition;
wherein the at least two latex polymers are present in the composition in a weight ratio ranging from 10:1 to 1:10; and wherein said composition produces a film having a Young's modulus ranging from 0.05 MPa to 5 GPa, and a strain, under stress at 0.5 MPa, that ranges up to 300%.

**2.** The hair styling composition of claim 1, wherein the at least two latex polymers are present in a combined amount ranging from 0.2% to 5% by weight, relative to the weight of the composition.

**3.** The hair styling composition of claim 1, wherein the at least two latex polymers are present in a combined amount ranging from 0.5% to 5% by weight, relative to the weight of the composition.

**4.** The hair styling composition of claim 1, wherein the at least two latex polymers are present in a combined amount ranging from 1% to 3% by weight, relative to the weight of the composition.

**5.** The hair styling composition of claim 1, wherein each of the at least two latex polymers is present in individual amounts ranging from 0.1% to 7.5% by weight, relative to the weight of the composition.

**6.** The hair styling composition of claim 1, wherein each of the at least two latex polymers is present in individual amounts ranging from 0.5% to 2.5% by weight, relative to the weight of the composition.

**7.** The hair styling composition of claim 1, wherein the at least two latex polymers are present in the composition in a weight ratio of 1:5 to 5:1.

**8.** The hair styling composition of claim 1, wherein the at least two latex polymers are present in the composition in a weight ratio of 1:3 to 3:1.

**9.** The hair styling composition of claim 1, wherein the at least two latex polymers are present in the composition in a weight ratio of 1:2 to 2:1.

**10.** The hair styling composition of claim 1, wherein the at least two latex polymers are present in the composition in a weight ratio of 1:1.

**11.** The hair styling composition of claim 1, wherein the film has a Young's modulus ranging from 80 MPa to 5 GPa and a strain, under stress at 0.5 MPa, ranging from 0.01% to less than 1%.

**12.** The hair styling composition of claim 11, wherein the weight ratio of polymer A to polymer B is 1:10 to 1:1.

**13.** The hair styling composition of claim 1, wherein the film has a Young's modulus ranging from 5 MPa to 100 MPa and a strain, under stress at 0.5 MPa, ranging from 0.5% to less than 20%.

**14.** The hair styling composition of claim 13, wherein the weight ratio of polymer A to polymer B is 3:1 to 10:1.

**15.** The hair styling composition of claim 1, wherein the film has a Young's modulus ranging from 0.05 MPa to 5 MPa and a strain, under stress at 0.5 MPa, ranging from 10% to 300%.

**16.** The hair styling composition of claim 15, wherein the weight ratio of polymer A to polymer B is 5:1 to 10:1.

**17.** The hair styling composition of claim 1, comprising at least one component chosen from coalescing agents and plasticizers, present in a total amount ranging from 0.1% to 20% by weight, relative to the weight of the composition.

**18.** The hair styling composition of claim 1, comprising at least one component chosen from coalescing agents and plasticizers, present in a total amount ranging from 0.1% to 1% by weight, relative to the weight of the composition.

**19.** The hair styling composition of claim 1, wherein the coalescing agents and plasticizers are chosen from glycol ethers, glycol esters, sucrose esters, propylene glycol ethers and propylene glycol esters.

**20.** The hair styling composition of claim 1, wherein the coalescing agents and plasticizers are chosen from propylene glycol dibenzoate, dipropylene glycol dibenzoate, and propylene glycol butyl ether.

**21.** The hair styling composition of claim 1, comprising at least one component chosen from thickening agents, present in a total amount ranging from 0.1% to 10% by weight, relative to the weight of the composition.

**22.** The hair styling composition of claim 1, comprising at least one component chosen from thickening agents, present in a total amount ranging from 1% to 3% by weight, relative to the weight of the composition.

**23.** The hair styling composition of claim 1, wherein the thickening agents are chosen from acrylates/C10-30 alkyl acrylate crosspolymer, xanthan gum, guar gum, hydroxypropyl guar, guar hydroxypropyl trimonium chloride, hydroxyethyl cellulose, hydroxypropyl cellulose and cetyl hydroxyethyl cellulose.

**24.** The hair styling composition of claim 1, further comprising at least one solvent.

**25.** A hair styling composition of claim 24, wherein the at least two latex polymers are comprised in an aqueous dispersion.

**26.** A method of styling the hair, said method comprising applying a composition to the hair, said composition comprising at least two latex polymers chosen from acrylate and polyurethane polymers and optionally at least one component chosen from coalescing agents, plasticizers, and/or thickening agents, wherein at least one latex polymer is a film-forming polymer;
wherein the composition comprises at least one latex polymer A and at least one latex polymer B chosen from:

(a) polymer A, having a Young's modulus ranging from 0.1 MPa to 10 MPa and a strain, under stress at 0.5 MPa, of at least 1%; and
(b) polymer B, having a Young's modulus ranging from 10 MPa to 6 GPa and a strain, under stress at 0.5 MPa, of less than 5%;

wherein the at least two latex polymers are comprised in an aqueous dispersion;
with the proviso that when the first latex polymer is chosen from acrylate polymers, the second latex polymer is chosen from polyurethane polymers; and when the first latex polymer is chosen from polyurethane polymers, the second latex polymer is chosen from acrylate polymers;
wherein the at least two latex polymers are present in a combined amount ranging from 0.1% to 8% by weight, relative to the weight of the composition;
wherein the at least two latex polymers are present in the composition in a weight ratio ranging from 10:1 to 1:10; and wherein said composition produces a film having a Young's modulus ranging from 0.05 MPa to 5 GPa, and a strain, under stress at 0.5 MPa, that ranges up to 300%.

**27.** The method of claim 26, further comprising a step of treating the hair with heat at a temperature ranging from 25°C to 250°C before, during, or after the application of said composition.

## Patentansprüche

**1.** Haarstylingzusammensetzung mit mindestens zwei Latexpolymeren, die aus Acrylat- und Polyurethanpolymeren ausgewählt sind, und optional mit mindestens einem Bestandteil, der aus Koaleszenzmitteln, Weichmachern und/oder Verdickungsmitteln ausgewählt ist, wobei mindestens ein Latexpolymer ein filmbildendes Polymer ist;
wobei die Zusammensetzung mindestens ein Latexpolymer A und mindestens ein Latexpolymer B umfasst, die ausgewählt sind aus:

(a) Polymer A, das einen Elastizitätsmodul im Bereich von etwa 0,1 MPa bis 10 MPa und, unter Belastung bei 0,5 MPa, eine Dehnung von mindestens 1% besitzt; und
(b) Polymer B, das einen Elastizitätsmodul im Bereich von 10 MPa bis 6 GPa und, unter Belastung bei 0,5 MPa, eine Dehnung von weniger als 5% besitzt;

wobei es sich bei den mindestens zwei Latexpolymeren, unabhängig voneinander oder zusammen, um dispergierte Partikel in einem wässrigen Dispersionsmedium handelt;
mit der Maßgabe, dass wenn das erste Latexpolymer aus Acrylatpolymeren ausgewählt ist, das zweite Latexpolymer aus Polyurethanpolymeren ausgewählt ist; und wenn das erste Latexpolymer aus Polyurethanpolymeren ausgewählt ist, das zweite Latexpolymer aus Acrylatpolymeren ausgewählt ist;
wobei die mindestens zwei Latexpolymere in einer kombinierten Menge im Bereich von 0,1 Gew.-% bis 8 Gew.-% vorliegen, bezogen auf das Gewicht der Zusammensetzung;
wobei die mindestens zwei Latexpolymere in der Zusammensetzung in einem Gewichtsverhältnis im Bereich von 10:1 bis 1:10 vorliegen; und
wobei die Zusammensetzung einen Film mit einem Elastizitätsmodul im Bereich von 0,05 MPa bis 5 GPa und, unter Belastung bei 0,5 MPa, einer Dehnung, die bis 300% reicht, erzeugt.

**2.** Haarstylingzusammensetzung nach Anspruch 1, wobei die mindestens zwei Latexpolymere in einer kombinierten Menge im Bereich von 0,2 Gew.-% bis 5 Gew.-% vorliegen, bezogen auf das Gewicht der Zusammensetzung.

**3.** Haarstylingzusammensetzung nach Anspruch 1, wobei die mindestens zwei Latexpolymere in einer kombinierten Menge im Bereich von 0,5 Gew.-% bis 5 Gew.-% vorliegen, bezogen auf das Gewicht der Zusammensetzung.

**4.** Haarstylingzusammensetzung nach Anspruch 1, wobei die mindestens zwei Latexpolymere in einer kombinierten Menge im Bereich von 1 Gew.-% bis 3 Gew.-% vorliegen, bezogen auf das Gewicht der Zusammensetzung.

**5.** Haarstylingzusammensetzung nach Anspruch 1, wobei jedes der mindestens zwei Latexpolymere in einer Einzelmenge im Bereich von 0,1 Gew.-% bis 7,5 Gew.-% vorliegt, bezogen auf das Gewicht der Zusammensetzung.

**6.** Haarstylingzusammensetzung nach Anspruch 1, wobei jedes der mindestens zwei Latexpolymere in einer Einzelmenge im Bereich von 0,5 Gew.-% bis 2,5 Gew.-% vorliegt, bezogen auf das Gewicht der Zusammensetzung.

**7.** Haarstylingzusammensetzung nach Anspruch 1, wobei die mindestens zwei Latexpolymere in der Zusammensetzung in einem Gewichtsverhältnis von 1:5 bis 5:1 vorliegen.

**8.** Haarstylingzusammensetzung nach Anspruch 1, wobei die mindestens zwei Latexpolymere in der Zusammensetzung in einem Gewichtsverhältnis von 1:3 bis 3:1 vorliegen.

**9.** Haarstylingzusammensetzung nach Anspruch 1, wobei die mindestens zwei Latexpolymere in der Zusammensetzung in einem Gewichtsverhältnis von 1:2 bis 2:1 vorliegen.

**10.** Haarstylingzusammensetzung nach Anspruch 1, wobei die mindestens zwei Latexpolymere in der Zusammensetzung in einem Gewichtsverhältnis von 1:1 vorliegen.

**11.** Haarstylingzusammensetzung nach Anspruch 1, wobei der Film einen Elastizitätsmodul im Bereich von 80 MPa bis 5 GPa und, unter Belastung bei 0,5 MPa, eine Dehnung im Bereich von 0,01% bis weniger als 1% besitzt.

**12.** Haarstylingzusammensetzung nach Anspruch 11, wobei das Gewichtsverhältnis von Polymer A zu Polymer B 1:10 bis 1:1 beträgt.

**13.** Haarstylingzusammensetzung nach Anspruch 1, wobei der Film einen Elastizitätsmodul im Bereich von 5 MPa bis 100 MPa und, unter Belastung bei 0,5 MPa, eine Dehnung im Bereich von 0,5% bis weniger als 20% besitzt.

**14.** Haarstylingzusammensetzung nach Anspruch 13, wobei das Gewichtsverhältnis von Polymer A zu Polymer B 3:1 bis 10:1 beträgt.

**15.** Haarstylingzusammensetzung nach Anspruch 1, wobei der Film einen Elastizitätsmodul im Bereich von 0,05 MPa bis 5 GPa und, unter Belastung bei 0,5 MPa, eine Dehnung im Bereich von 10% bis 300% besitzt.

**16.** Haarstylingzusammensetzung nach Anspruch 15, wobei das Gewichtsverhältnis von Polymer A zu Polymer B 5:1 bis 10:1 beträgt.

**17.** Haarstylingzusammensetzung nach Anspruch 1, umfassend mindestens einen Bestandteil, der aus Koaleszenzmitteln und Weichmachern ausgewählt ist, die in einer Gesamtmenge im Bereich von 0,1 Gew.-% bis 20 Gew.-% vorliegen, bezogen auf das Gewicht der Zusammensetzung.

**18.** Haarstylingzusammensetzung nach Anspruch 1, umfassend mindestens einen Bestandteil, der aus Koaleszenzmitteln und Weichmachern ausgewählt ist, die in einer Gesamtmenge im Bereich von 0,1 Gew.-% bis 1 Gew.-% vorliegen, bezogen auf das Gewicht der Zusammensetzung.

**19.** Haarstylingzusammensetzung nach Anspruch 1, wobei die Koaleszenzmittel und Weichmacher aus Glycolethern, Glycolestern, Saccharoseestern, Propylenglycolethern und Propylenglycolestern ausgewählt sind.

**20.** Haarstylingzusammensetzung nach Anspruch 1, wobei die Koaleszenzmittel und Weichmacher aus Propylenglycoldibenzoat, Dipropylenglycoldibenzoat und Propylenglycolbutylether ausgewählt sind.

**21.** Haarstylingzusammensetzung nach Anspruch 1, umfassend mindestens einen Bestandteil, der aus Verdickungsmitteln ausgewählt ist, die in einer Gesamtmenge im Bereich von 0,1 Gew.-% bis 10 Gew.-% vorliegen, bezogen auf das Gewicht der Zusammensetzung.

**22.** Haarstylingzusammensetzung nach Anspruch 1, umfassend mindestens einen Bestandteil, der aus Verdickungsmitteln ausgewählt ist, die in einer Gesamtmenge im Bereich von 1 Gew.-% bis 3 Gew.-% vorliegen, bezogen auf das Gewicht der Zusammensetzung.

**23.** Haarstylingzusammensetzung nach Anspruch 1, wobei die Verdickungsmittel aus Acrylat/$C_{10-30}$-Alkylacrylat-Crosspolymer, Xanthangummi, Guargummi, Hydroxypropylguar, Guarhydroxypropyltrimoniumchlorid, Hydroxyethylcellulose, Hydroxypropylcellulose und Cetylhydroxyethylcellulose ausgewählt sind.

**24.** Haarstylingzusammensetzung nach Anspruch 1, ferner umfassend mindestens ein Lösungsmittel.

**25.** Haarstylingzusammensetzung nach Anspruch 24, wobei die mindestens zwei Latexpolymere in einer wässrigen Dispersion enthalten sind.

**26.** Verfahren zum Stylen des Haares, wobei bei dem Verfahren eine Zusammensetzung auf das Haar aufgetragen wird, wobei die Zusammensetzung mindestens zwei Latexpolymere umfasst, die aus Acrylat- und Polyurethanpolymeren ausgewählt sind, und optional mindestens einen Bestandteil, der aus Koaleszenzmitteln, Weichmachern und/oder Verdickungsmitteln ausgewählt ist, wobei mindestens ein Latexpolymer ein filmbildendes Polymer ist;
wobei die Zusammensetzung mindestens ein Latexpolymer A und mindestens ein Latexpolymer B umfasst, die ausgewählt sind aus:

(a) Polymer A, das einen Elastizitätsmodul im Bereich von 0,1 MPa bis 10 MPa und, unter Belastung bei 0,5 MPa, eine Dehnung von mindestens 1% besitzt; und
(b) Polymer B, das einen Elastizitätsmodul im Bereich von 10 MPa bis 6 GPa und, unter Belastung bei 0,5 MPa, eine Dehnung von weniger als 5% besitzt;

wobei die mindestens zwei Latexpolymere in einer wässrigen Dispersion enthalten sind;
mit der Maßgabe, dass wenn das erste Latexpolymer aus Acrylatpolymeren ausgewählt ist, das zweite Latexpolymer aus Polyurethanpolymeren ausgewählt ist; und wenn das erste Latexpolymer aus Polyurethanpolymeren ausgewählt ist, das zweite Latexpolymer aus Acrylatpolymeren ausgewählt ist;
wobei die mindestens zwei Latexpolymere in einer kombinierten Menge im Bereich von 0,1 Gew.-% bis 8 Gew.-% vorliegen, bezogen auf das Gewicht der Zusammensetzung;
wobei die mindestens zwei Latexpolymere in der Zusammensetzung in einem Gewichtsverhältnis im Bereich von 10:1 bis 1:10 vorliegen; und
wobei die Zusammensetzung einen Film mit einem Elastizitätsmodul im Bereich von 0,05 MPa bis 5 GPa und, unter Belastung bei 0,5 MPa, einer Dehnung, die bis 300% reicht, erzeugt.

**27.** Verfahren nach Anspruch 26, ferner umfassend einen Schritt, bei dem das Haar vor, während oder nach dem Aufbringen der Zusammensetzung mit Wärme bei einer Temperatur im Bereich von 25°C bis 250°C behandelt wird.

## Revendications

1. Composition de coiffage comprenant au moins deux polymères de latex choisis parmi des polymères d'acrylate et de polyuréthane et éventuellement au moins un composant choisi parmi des agents coalescents, des plastifiants, et/ou des agents épaississants, dans laquelle au moins un polymère de latex est un polymère filmogène ;
dans laquelle la composition comprend au moins un polymère de latex A et au moins un polymère de latex B choisis parmi :

    (a) un polymère A, ayant un module de Young dans la plage de 0,1 MPa à 10 MPa et un effort, sous contrainte à 0,5 MPa, d'au moins 1 % ; et
    (b) un polymère B, ayant un module de Young dans la plage de 10 MPa à 6 GPa et un effort, sous contrainte à 0,5 MPa, de moins de 5 % ;

    dans laquelle les au moins deux polymères de latex sont, indépendamment ou conjointement, des particules dispersées dans un milieu de dispersion aqueux ;
à condition que lorsque le premier polymère de latex est choisi parmi des polymères d'acrylate, le second polymère de latex soit choisi parmi des polymères de polyuréthane ; et lorsque le premier polymère de latex est choisi parmi des polymères de polyuréthane, le second polymère de latex soit choisi parmi des polymères d'acrylate ;
dans laquelle les au moins deux polymères de latex sont présents dans une quantité combinée dans la plage de 0,1 % à 8 % en poids, par rapport au poids de la composition ;
dans laquelle les au moins deux polymères de latex sont présents dans la composition dans un rapport en poids dans la plage de 10:1 à 1:10 ; et
dans laquelle ladite composition produit un film ayant un module de Young dans la plage de 0,05 MPa à 5 GPa, et un effort, sous contrainte à 0,5 MPa, qui va jusqu'à 300 %.

2. Composition de coiffage selon la revendication 1, dans laquelle les au moins deux polymères de latex sont présents dans une quantité combinée dans la plage de 0,2 % à 5 % en poids, par rapport au poids de la composition.

3. Composition de coiffage selon la revendication 1, dans laquelle les au moins deux polymères de latex sont présents dans une quantité combinée dans la plage de 0,5 % à 5 % en poids, par rapport au poids de la composition.

4. Composition de coiffage selon la revendication 1, dans laquelle les au moins deux polymères de latex sont présents dans une quantité combinée dans la plage de 1 % à 3 % en poids, par rapport au poids de la composition.

5. Composition de coiffage selon la revendication 1, dans laquelle chacun des au moins deux polymères de latex est présent dans des quantités individuelles dans la plage de 0,1% à 7,5% en poids, par rapport au poids de la composition.

6. Composition de coiffage selon la revendication 1, dans laquelle chacun des au moins deux polymères de latex est présent dans des quantités individuelles dans la plage de 0,5 % à 2,5 % en poids, par rapport au poids de la composition.

7. Composition de coiffage selon la revendication 1, dans laquelle les au moins deux polymères de latex sont présents dans la composition dans un rapport en poids de 1:5 à 5:1.

8. Composition de coiffage selon la revendication 1, dans laquelle les au moins deux polymères de latex sont présents dans la composition dans un rapport en poids de 1:3 à 3:1.

9. Composition de coiffage selon la revendication 1, dans laquelle les au moins deux polymères de latex sont présents dans la composition dans un rapport en poids de 1:2 à 2:1.

10. Composition de coiffage selon la revendication 1, dans laquelle les au moins deux polymères de latex sont présents dans la composition dans un rapport en poids de 1:1.

11. Composition de coiffage selon la revendication 1, dans laquelle le film a un module de Young dans la plage de 80 MPa à 5 GPa et un effort, sous contrainte à 0,5 MPa, dans la plage de 0,01 % à moins de 1 %.

12. Composition de coiffage selon la revendication 11, dans laquelle le rapport en poids entre le polymère A et le

polymère B est de 1:10 à 1:1.

**13.** Composition de coiffage selon la revendication 1, dans laquelle le film a un module de Young dans la plage de 5 MPa à 100 MPa et un effort, sous contrainte à 0,5 MPa, dans la plage de 0,5 % à moins de 20 %.

**14.** Composition de coiffage selon la revendication 13, dans laquelle le rapport en poids entre le polymère A et le polymère B est de 3:1 à 10:1.

**15.** Composition de coiffage selon la revendication 1, dans laquelle le film a un module de Young dans la plage de 0,05 MPa à 5 MPa et un effort, sous contrainte à 0,5 MPa, dans la plage de 10 % à 300 %.

**16.** Composition de coiffage selon la revendication 15, dans laquelle le rapport en poids entre le polymère A et le polymère B est de 5:1 à 10:1.

**17.** Composition de coiffage selon la revendication 1, comprenant au moins un composant choisi parmi des agents coalescents et des plastifiants, présents dans une quantité totale dans la plage de 0,1 % à 20 % en poids, par rapport au poids de la composition.

**18.** Composition de coiffage selon la revendication 1, comprenant au moins un composant choisi parmi des agents coalescents et des plastifiants, présents dans une quantité totale dans la plage de 0,1 % à 1 % en poids, par rapport au poids de la composition.

**19.** Composition de coiffage selon la revendication 1, dans laquelle les agents coalescents et plastifiants sont choisis parmi les éthers de glycol, les esters de glycol, les esters de saccharose, les éthers de propylène glycol et les esters de propylène glycol.

**20.** Composition de coiffage selon la revendication 1, dans laquelle les agents coalescents et plastifiants sont choisis parmi le dibenzoate de propylène glycol, le dibenzoate de dipropylène glycol, et le butyl éther de propylène glycol.

**21.** Composition de coiffage selon la revendication 1, comprenant au moins un composant choisi parmi des agents épaississants, présents dans une quantité totale dans la plage de 0,1 % à 10 % en poids, par rapport au poids de la composition.

**22.** Composition de coiffage selon la revendication 1, comprenant au moins un composant choisi parmi des agents épaississants, présents dans une quantité totale dans la plage de 1 % à 3 % en poids, par rapport au poids de la composition.

**23.** Composition de coiffage selon la revendication 1, dans laquelle les agents épaississants sont choisis parmi un polymère réticulé d'acrylates/acrylate d'alkyle en $C_{10}$ à $C_{30}$, la gomme de xanthane, la gomme de guar, l'hydroxypropyl guar, le chlorure d'hydroxypropyl trimonium guar, l'hydroxyéthyl cellulose, l'hydroxypropyl cellulose et la cétyl hydroxyéthyl cellulose.

**24.** Composition de coiffage selon la revendication 1, comprenant en outre au moins un solvant.

**25.** Composition de coiffage selon la revendication 24, dans laquelle les au moins deux polymères de latex sont compris dans une dispersion aqueuse.

**26.** Procédé de coiffage des cheveux, ledit procédé comprenant l'application d'une composition sur les cheveux, ladite composition comprenant au moins deux polymères de latex choisis parmi des polymères d'acrylate et de polyuréthane et éventuellement au moins un composant choisi parmi des agents coalescents, des plastifiants, et/ou des agents épaississants, dans lequel au moins un polymère de latex est un polymère filmogène ; dans lequel la composition comprend au moins un polymère de latex A et au moins un polymère de latex B choisis parmi :

(a) un polymère A, ayant un module de Young dans la plage de 0,1 MPa à 10 MPa et un effort, sous contrainte à 0,5 MPa, d'au moins 1 % ; et
(b) un polymère B, ayant un module de Young dans la plage de 10 MPa à 6 GPa et un effort, sous contrainte à 0,5 MPa, de moins de 5 % ;

dans lequel les au moins deux polymères de latex sont compris dans une dispersion aqueuse ;
à condition que lorsque le premier polymère de latex est choisi parmi des polymères d'acrylate, le second polymère de latex soit choisi parmi des polymères de polyuréthane ; et lorsque le premier polymère de latex est choisi parmi des polymères de polyuréthane, le second polymère de latex soit choisi parmi des polymères d'acrylate ;
dans lequel les au moins deux polymères de latex sont présents dans une quantité combinée dans la plage de 0,1 % à 8 % en poids, par rapport au poids de la composition ; dans lequel les au moins deux polymères de latex sont présents dans la composition dans un rapport en poids dans la plage de 10:1 à 1:10 ; et
dans lequel ladite composition produit un film ayant un module de Young dans la plage de 0,05 MPa à 5 GPa, et un effort, sous contrainte à 0,5 MPa, qui va jusqu'à 300 %.

**27.** Procédé selon la revendication 26, comprenant en outre une étape de traitement des cheveux avec de la chaleur à une température dans la plage de 25 °C à 250 °C avant, pendant, ou après l'application de ladite composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6126929 A
- US 4710374 A
- US 7740832 B
- US 4798721 A
- US 20050089490 A1
- US 20070224140 A1
- FR 2968978 A
- FR 2898050 A
- US 20090297467 A
- US 2009035335 A1
- WO 2011137338 A2
- US 20040071646 A
- US 20030064045 A1
- US 20070286833 A1
- US 20090317432 A
- EP 1082953 A
- WO 11056332 A
- US 20100028284 A1
- US 2006134043 A
- US 7651693 B
- US 6214328 B
- US 5441728 A
- FR 2834458 A
- US 3383351 A
- US 3304273 A
- US 3523095 A
- US 3110695 A
- DE 1152536
- US 3412054 A
- EP 0216479 A
- US 3915921 A
- US 4509949 A
- FR 2633940
- EP 898960 A
- EP 898958 A


### Non-patent literature cited in the description


- Model TA-XTPlus, Texture Technologies Corporation) equipped with a hair mounting accessory as described. *J. Cosmet. Sci.,* November 2002, vol. 53, 345-362